# EUROPEAN PATENT APPLICATION

(11) **EP 4 596 538 A1**
(43) Date of publication of application: **06.08.2025**
(21) Application number: 23871006.5
(22) Date of filing: 28.09.2023
(51) Int. Cl.: C07D 401/14, C07D 403/14, A61P 35/00, A61K 31/4725

(54) **PHARMACEUTICALLY ACCEPTABLE SALT AND CRYSTAL FORM OF TETRAHYDRONAPHTHALENE DERIVATIVE, AND PREPARATION METHOD**

(30) Priority: 29.09.2022 CN 202211197780
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: JIA, Lina, Lianyungang, Jiangsu 222047 (CN); YANG, Junran, Lianyungang, Jiangsu 222047 (CN); DU, Zhenxing, Lianyungang, Jiangsu 222047 (CN); WANG, Lin, Shanghai 200245 (CN); SHAO, Qiyun, Shanghai 200245 (CN); FENG, Jun, Shanghai 200245 (CN)
(74) Representative: dompatent von Kreisler Selting Werner - Partnerschaft von Patent- und Rechtsanwälten mbB
(86) International application number: PCT/CN2023/122461
(87) International publication number: WO 2024/067781

(57) **Abstract**

The present invention relates to a pharmaceutically acceptable salt and a crystal form of a tetrahydronaphthalene derivative, and a preparation method. Specifically, the present disclosure provides a pharmaceutically acceptable salt and a crystal form of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, and a preparation method therefor. The corresponding salt has good stability and can be better used for clinical treatment.

## Description

The present application claims priority to Chinese Patent Application No. 2022111977804 filed on September 29, 2022, which is incorporated herein by reference in its entirety.

### TECHNICAL FIELD

The present disclosure pertains to the field of pharmaceutics and relates to pharmaceutically acceptable salts and crystal forms of a tetrahydronaphthalene derivative.

### BACKGROUND

Protein proteolysis-targeting chimeras (PROTACs) are hybrid bifunctional small-molecule compounds. Their structures contain two different ligands: an E3 ubiquitin ligase ligand and a ligand that binds to a target protein. The two ligands are linked by a linker arm. PROTACs draw close the target protein and the E3 ubiquitin ligase in cells to form target protein-PROTAC-E3 ternary complexes. Then E3 ubiquitin ligase labels the target protein with a ubiquitinated protein tag, initiating a powerful ubiquitination hydrolysis process in cells in which the target protein is specifically degraded via the ubiquitin-proteasome pathway. PROTACs have unique advantages over traditional small-molecule inhibitors: 1. PROTACs do not need to highly strongly bind to the target protein for a long time, and the degradation of the target protein is similar to catalysis: they can bind and degrade the target protein cyclically, such that the systemic drug exposure and the occurrence of toxic and side effects are reduced. 2. After being degraded, the target protein needs to be re-synthesized to recover its function; therefore, degrading the target protein exhibits a more efficient and lasting anti-tumor effect than inhibiting its activity and will not lead to drug resistance caused by mutation of the target protein. 3. PROTACs also have therapeutic potentials for targets that are now considered undruggable, such as transcription factors, scaffold proteins, and regulatory proteins.

The discovery of cereblon (CRBN)-type E3 ligase ligands is associated with studies on the mechanism of action of thalidomide. In 2010, a study on the toxicity of thalidomide revealed that the *in vivo* binding of thalidomide to CRBN may be the cause of the teratogenicity of thalidomide (Science, 2010, 327, 1345). Subsequent studies revealed that thalidomide and its derivatives can be used as anti-inflammatory drugs, anti-angiogenesis drugs, and anti-cancer drugs. Among them, lenalidomide and pomalidomide are much safer in that they have significantly lower teratogenicity. A further study has shown that lenalidomide acts by degrading two special B-cell transcription factors: Ikaros family zinc finger proteins 1 and 3 (IKZF1 and IKZF3). This study revealed the mechanism of action of thalidomide and its derivatives: they bind to CRBN-type E3 ubiquitin ligase protein complexes, thereby degrading the target protein. On this basis, CRBN ligands have been widely used in the preparation of protein degraders, and a range of PROTAC molecules based on CRBN ligands have been developed. PCT/CN2022/083597 discloses a class of novel tetrahydronaphthalene derivatives and demonstrates the use of compound I, the chemical name of which is (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, as an estrogen receptor degrader in the treatment of estrogen receptor-mediated or -dependent diseases.

The structure of a crystal form used as a pharmaceutically active ingredient often affects the chemical and physical stabilities of the drug. Changes in crystallization and storage conditions may cause changes in the crystal structure of the compound and sometimes the generation of other crystal forms. Generally, drug products of amorphous forms do not have a regular crystal structure and often have other defects, such as having relatively poor product stability, being relatively difficult to filter, being prone to agglomeration, and having poor flowability. Therefore, the research on pharmaceutically acceptable salts and crystal forms of (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione is of great significance for the development of drugs that are suitable for industrial production and have good biological activity.

### SUMMARY

In one aspect, the present disclosure provides a crystal form A of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione.

In some embodiments, an X-ray powder diffraction pattern expressed in terms of 2*θ* angles, which are diffraction angles, of the crystal form A has characteristic peaks at 14.2, 15.3, 16.1, 17.4, and 19.1.

In some other embodiments, the X-ray powder diffraction pattern expressed in terms of 2*θ* angles, which are diffraction angles, of the crystal form A has characteristic peaks at 14.2, 15.3, 16.1, 17.4, 19.1, 20.0, 20.9, and 22.3.

In some other embodiments, the X-ray powder diffraction pattern expressed in terms of 2*θ* angles, which are diffraction angles, of the crystal form A has characteristic peaks at 5.0, 6.0, 14.2, 15.3, 16.1, 17.4, 19.1, 20.0, 20.9, 22.3, 24.8, and 26.9.

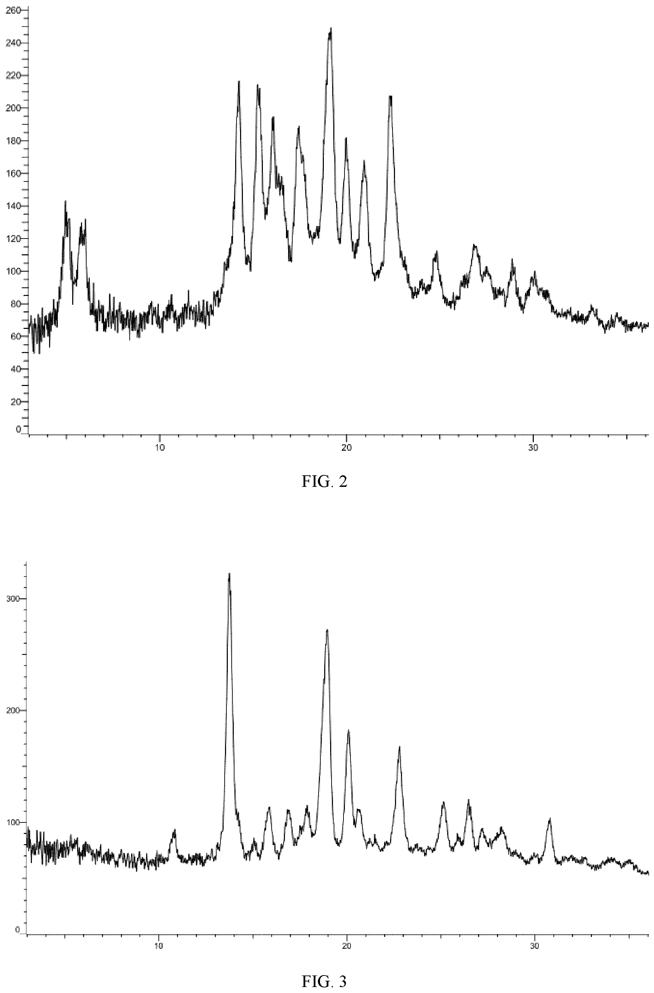

In some other embodiments, the X-ray powder diffraction pattern expressed in terms of 2*θ* angles, which are diffraction angles, of the crystal form A is shown in FIG. 2.

In another aspect, the present disclosure provides a pharmaceutically acceptable salt of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein the pharmaceutically acceptable salt is selected from the group consisting of hydrochloride, maleate, fumarate, L-tartrate, succinate, D-malate, L-malate, sulfate, phosphate, and citrate.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to acid is 3:1-1:3, including, but not limited to, 3:1, 2:1, 1:1, 1:2, and 1:3.

In another embodiment, the chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to acid is 2:1-1:2.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to hydrogen chloride is 1:1 or 1:2.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to maleic acid is 1:1.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to fumaric acid is 1:1 or 1:2.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to L-tartaric acid is 1:1 or 2:1.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to succinic acid is 1:1.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to D-malic acid is 2:1 or 1:1.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to L-malic acid is 2:1 or 1:1.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to sulfuric acid is 1:1 or 1:2.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to phosphoric acid is 1:1.

In an optional embodiment, a chemical ratio of the compound (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to citric acid is 1:1.

The present disclosure further provides a preparation method for a pharmaceutically acceptable salt of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, the method comprising a step of reacting (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione with an acid, wherein the acid is selected from the group consisting of hydrochloric acid, maleic acid, fumaric acid, L-tartaric acid, succinic acid, D-malic acid, L-malic acid, sulfuric acid, phosphoric acid, and citric acid.

Solvents used for salt formation in the present disclosure are selected from the group consisting of, but are not limited to, acetonitrile, acetone, tetrahydrofuran, ethanol, methanol, 1,4-dioxane, ethanol/ethyl acetate, methanol, ethanol/water, tetrahydrofuran/ethanol, dichloromethane/ethanol, and methyl tert-butyl ether/ethanol.

Further, in an optional embodiment, the method for preparing the aforementioned pharmaceutically acceptable salt further comprises such a step as crystallization, filtration, washing, or drying.

In another aspect, the present disclosure further provides a hydrochloride crystal form I of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the hydrochloride crystal form I has characteristic peaks at 13.8, 15.9, 19.0, 20.1, and 22.8.

In some other embodiments, the X-ray powder diffraction pattern expressed in terms of 2*θ* angles, which are diffraction angles, of the hydrochloride crystal form I has characteristic peaks at 13.8, 15.9, 16.9, 17.9, 19.0, 20.1, 20.6, and 22.8.

In some other embodiments, the X-ray powder diffraction pattern expressed in terms of 2*θ* angles, which are diffraction angles, of the hydrochloride crystal form I has characteristic peaks at 10.8, 13.8, 15.9, 16.9, 17.9, 19.0, 20.1, 20.6, 22.8, 25.2, and 26.5.

In some other embodiments, the X-ray powder diffraction pattern expressed in terms of 2*θ* angles, which are diffraction angles, of the hydrochloride crystal form I is shown in FIG. 3.

The present disclosure further provides a hydrochloride crystal form II of the compound (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the hydrochloride crystal form II has characteristic peaks at 13.2, 17.1, 19.7, 20.6, 22.8, and 25.0.

In some embodiments, the X-ray powder diffraction pattern expressed in terms of 2*θ* angles, which are diffraction angles, of the hydrochloride crystal form II has characteristic peaks at 10.4, 13.2, 14.5, 15.1, 17.1, 19.7, 20.6, 22.8, 25.0, and 26.1.

In some embodiments, the X-ray powder diffraction pattern expressed in terms of 2*θ* angles, which are diffraction angles, of the hydrochloride crystal form II has characteristic peaks at 5.6, 10.4, 13.2, 13.8, 14.5, 15.1, 17.1, 19.7, 20.6, 22.8, 23.3, 25.0, and 26.1.

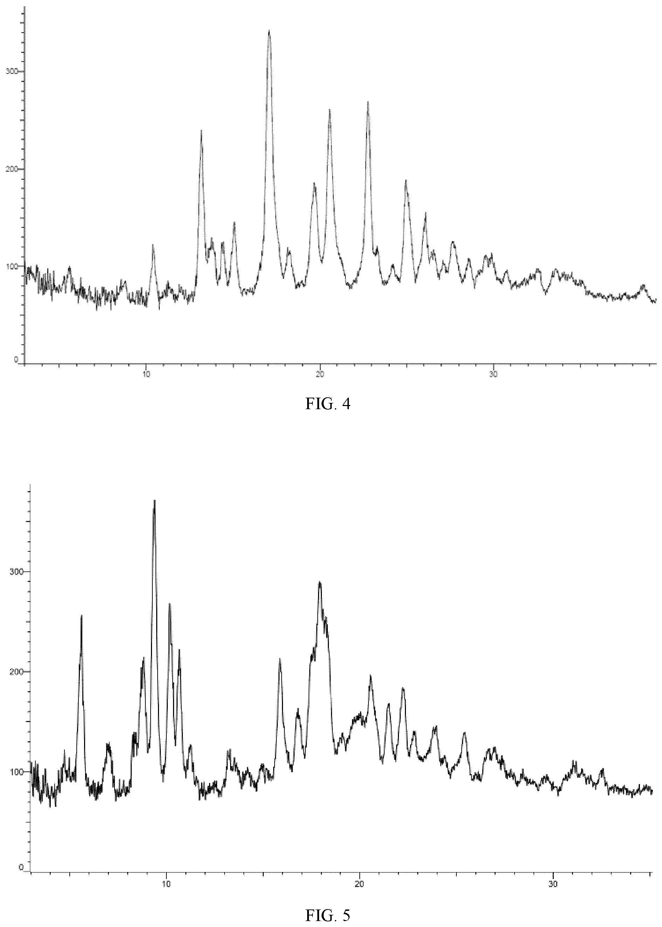

In some other embodiments, the X-ray powder diffraction pattern expressed in terms of 2*θ* angles, which are diffraction angles, of the hydrochloride crystal form I is shown in FIG. 4.

The present disclosure further provides a maleate crystal form I of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the maleate crystal form I has characteristic peaks at 5.6, 8.8, 9.4, 10.2, 10.7, and 18.0.

In some embodiments, the X-ray powder diffraction pattern expressed in terms of 2*θ* angles, which are diffraction angles, of the maleate crystal form I has characteristic peaks at 5.6, 7.0, 8.8, 9.4, 10.2, 10.7, 15.9, 18.0, 20.6, and 22.3.

In some embodiments, the X-ray powder diffraction pattern expressed in terms of 2*θ* angles, which are diffraction angles, of the maleate crystal form I has characteristic peaks at 5.6, 7.0, 8.4, 8.8, 9.4, 10.2, 10.7, 11.3, 15.9, 16.8, 18.0, 20.6, 21.5, 22.3, and 23.9.

In some embodiments, the X-ray powder diffraction pattern expressed in terms of 2*θ* angles, which are diffraction angles, of the maleate crystal form I is shown in FIG. 5.

The present disclosure further provides a fumarate crystal form I of the compound (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the fumarate crystal form I has characteristic peaks at 6.8, 9.6, 10.5, 17.6, 18.2, and 21.1.

In some embodiments, the X-ray powder diffraction pattern expressed in terms of 2*θ* angles, which are diffraction angles, of the fumarate crystal form I has characteristic peaks at 5.7, 6.8, 9.6, 10.5, 16.0, 17.6, 18.2, 19.8, and 21.1.

In some embodiments, the X-ray powder diffraction pattern expressed in terms of 2*θ* angles, which are diffraction angles, of the fumarate crystal form I has characteristic peaks at 5.7, 6.8, 8.9, 9.6, 10.5, 11.3, 16.0, 17.6, 18.2, 19.8, 21.1, and 22.2.

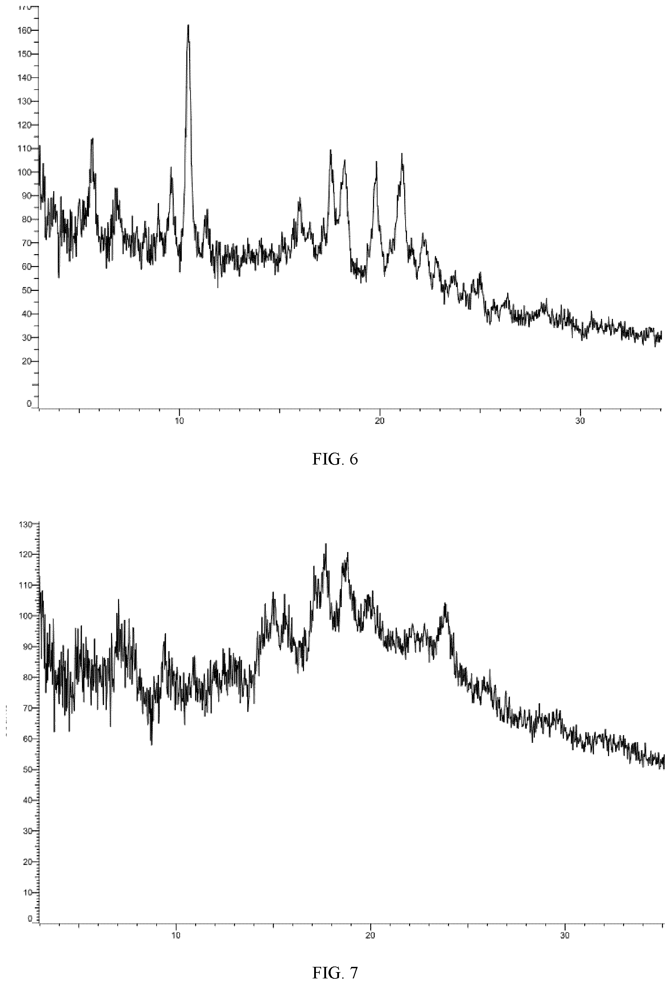

In some embodiments, the X-ray powder diffraction pattern expressed in terms of 2*θ* angles, which are diffraction angles, of the fumarate crystal form I is shown in FIG. 6.

The present disclosure further provides a fumarate crystal form II of the compound (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the fumarate crystal form II has characteristic peaks at 7.0, 9.4, 15.0, 17.1, 17.7, and 18.7.

In some embodiments, the X-ray powder diffraction pattern expressed in terms of 2*θ* angles, which are diffraction angles, of the fumarate crystal form II has characteristic peaks at 7.0, 9.4, 10.9, 14.6, 15.0, 17.1, 17.7, 18.7, 19.9, and 23.9.

In some embodiments, the X-ray powder diffraction pattern expressed in terms of 2*θ* angles, which are diffraction angles, of the fumarate crystal form II is shown in FIG. 7.

The present disclosure further provides a fumarate crystal form III of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the fumarate crystal form III has characteristic peaks at 5.1, 9.6, 10.4, 17.9, 18.5, and 20.5.

In some embodiments, the X-ray powder diffraction pattern expressed in terms of 2*θ* angles, which are diffraction angles, of the fumarate crystal form III has characteristic peaks at 5.1, 6.0, 9.6, 10.4, 17.9, 18.5, 19.4, 20.5, and 23.2.

In some embodiments, the X-ray powder diffraction pattern expressed in terms of 2*θ* angles, which are diffraction angles, of the fumarate crystal form III has characteristic peaks at 5.1, 6.0, 7.1, 9.6, 10.4, 11.3, 17.9, 18.5, 19.4, 20.5, and 23.2.

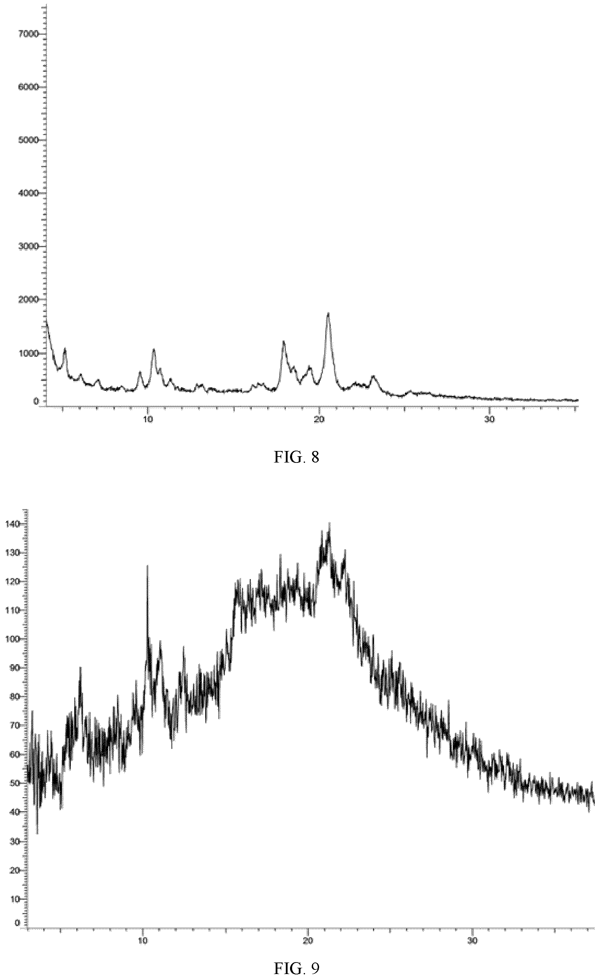

In some embodiments, the X-ray powder diffraction pattern expressed in terms of 2*θ* angles, which are diffraction angles, of the fumarate crystal form III is shown in FIG. 8.

The present disclosure further provides a fumarate crystal form IV of the compound (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the fumarate crystal form IV has characteristic peaks at 6.2, 10.3, 11.1, 12.5, 21.3, and 22.2.

In some embodiments, the X-ray powder diffraction pattern expressed in terms of 2*θ* angles, which are diffraction angles, of the fumarate crystal form IV is shown in FIG. 9.

The present disclosure further provides a fumarate crystal form V of the compound (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the fumarate crystal form V has characteristic peaks at 10.5, 17.1, 18.6, 20.1, 21.0, and 23.7.

In some embodiments, the X-ray powder diffraction pattern expressed in terms of 2*θ* angles, which are diffraction angles, of the fumarate crystal form V has characteristic peaks at 5.3, 9.9, 10.5, 11.6, 16.0, 17.1, 18.6, 20.1, 21.0, and 23.7.

In some embodiments, the X-ray powder diffraction pattern expressed in terms of 2*θ* angles, which are diffraction angles, of the fumarate crystal form V has characteristic peaks at 5.3, 6.2, 9.9, 10.5, 11.6, 15.6, 16.0, 17.1, 18.6, 20.1, 21.0, 22.7, 23.2, and 23.7.

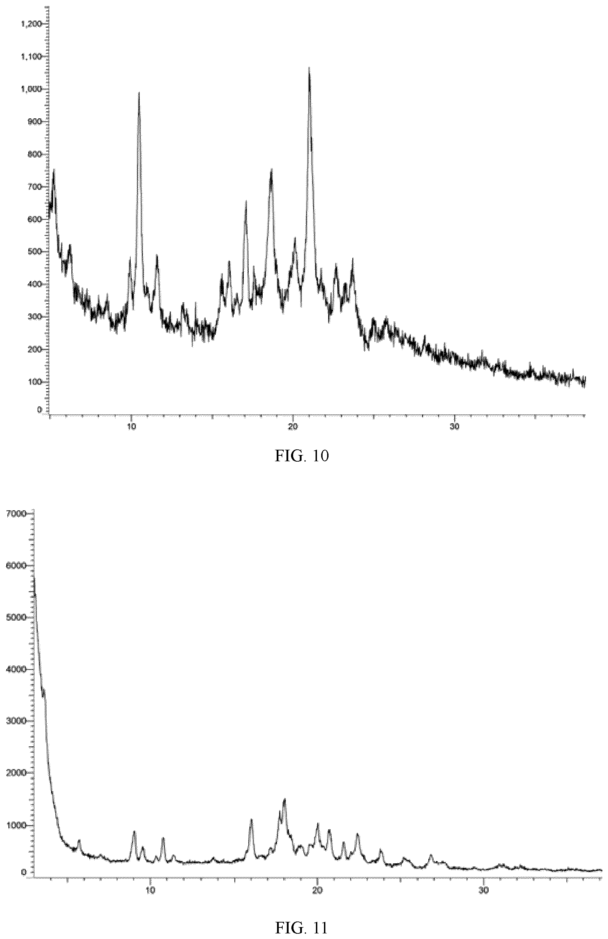

In some embodiments, the X-ray powder diffraction pattern expressed in terms of 2*θ* angles, which are diffraction angles, of the fumarate crystal form V is shown in FIG. 10.

The present disclosure further provides an L-tartrate crystal form I of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the L-tartrate crystal form I has characteristic peaks at 9.1, 16.0, 17.7, 18.0, 20.0, and 20.7.

In some embodiments, the X-ray powder diffraction pattern expressed in terms of 2*θ* angles, which are diffraction angles, of the L-tartrate crystal form I has characteristic peaks at 9.1, 9.5, 10.8, 16.0, 17.7, 18.0, 20.0, 20.7, 21.6, and 22.4.

In some embodiments, the X-ray powder diffraction pattern expressed in terms of 2*θ* angles, which are diffraction angles, of the L-tartrate crystal form I has characteristic peaks at 5.7, 9.1, 9.5, 10.8, 16.0, 17.2, 17.7, 18.0, 19.6, 20.0, 20.7, 21.6, 22.4, and 23.9.

In some embodiments, the X-ray powder diffraction pattern expressed in terms of 2*θ* angles, which are diffraction angles, of the L-tartrate crystal form I is shown in FIG. 11.

The present disclosure further provides a succinate crystal form I of the compound (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the succinate crystal form I has characteristic peaks at 9.5, 16.0, 17.6, 18.0, 19.9, 20.7, and 22.3.

In some embodiments, the X-ray powder diffraction pattern expressed in terms of 2*θ* angles, which are diffraction angles, of the succinate crystal form I has characteristic peaks at 9.0, 9.5, 10.3, 16.0, 17.6, 18.0, 19.9, 20.7, 21.5, and 22.3.

In some embodiments, the X-ray powder diffraction pattern expressed in terms of 2*θ* angles, which are diffraction angles, of the succinate crystal form I has characteristic peaks at 5.6, 6.9, 9.0, 9.5, 10.3, 10.7, 13.7, 16.0, 17.6, 18.0, 19.9, 20.7, 21.5, and 22.3.

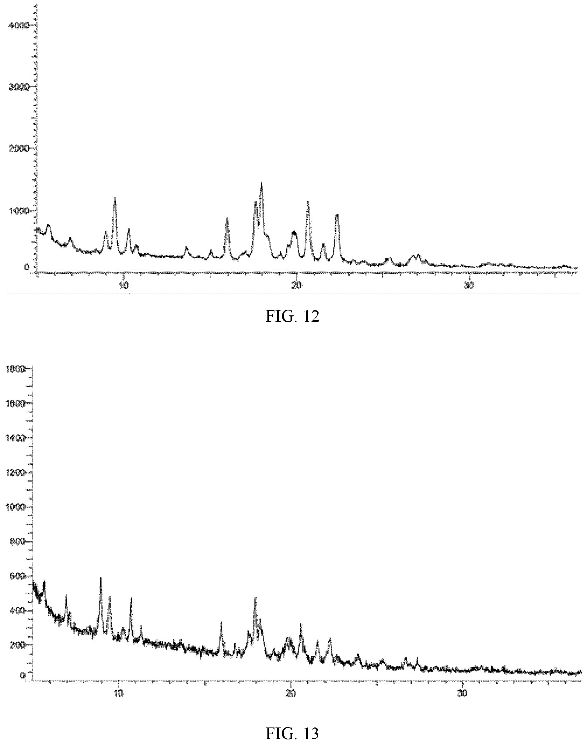

In some embodiments, the X-ray powder diffraction pattern expressed in terms of 2*θ* angles, which are diffraction angles, of the succinate crystal form I is shown in FIG. 12.

The present disclosure further provides a D-malate crystal form I of the compound (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione; an X-ray powder diffraction pattern expressed in terms of 2*θ* angles, which are diffraction angles, of the D-malate crystal form I has characteristic peaks at 9.0, 9.5, 10.7, 15.9, 17.9, 18.2, and 20.6.

In some embodiments, the X-ray powder diffraction pattern expressed in terms of 2*θ* angles, which are diffraction angles, of the D-malate crystal form I has characteristic peaks at 7.0, 9.0, 9.5, 10.7, 15.9, 17.9, 18.2, 20.0, 20.6, and 22.3.

In some embodiments, the X-ray powder diffraction pattern expressed in terms of 2*θ* angles, which are diffraction angles, of the D-malate crystal form I has characteristic peaks at 5.6, 7.0, 9.0, 9.5, 10.7, 15.9, 17.6, 17.9, 18.2, 20.0, 20.6, 21.5, and 22.3.

In some embodiments, the X-ray powder diffraction pattern expressed in terms of 2*θ* angles, which are diffraction angles, of the D-malate crystal form I is shown in FIG. 13.

Further, for the crystal forms of the compound (S)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione of the present disclosure, the 2*θ* angles have a margin of error of ±0.2.

In another aspect, the present disclosure provides a preparation method for the crystal form A of the compound (*S*)-3-(5-(4-((1-(4-((1*R*,2*R*)-6-hydroxy-2-isobuty,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in 50% ethanol/ethyl acetate, adding a hydrochloric acid solution, stirring, filtering, adding a phosphate buffer, and stirring for crystallization, or the following steps: adding hydrochloride of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to a phosphate buffer and stirring.

In some embodiments, the phosphate buffer is selected from the group consisting of, but is not limited to, sodium dihydrogen phosphate and disodium hydrogen phosphate. In some embodiments, the phosphate buffer has a pH of 4-8.

The present disclosure further provides a method for preparing the hydrochloride crystal form I of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, the method comprising: 1) dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in a solvent (1) and adding hydrochloric acid, and 2) stirring, wherein the solvent (1) is selected from the group consisting of ethanol/ethyl acetate, methanol, ethanol/water, tetrahydrofuran/ethanol, dichloromethane/ethanol, and methyl tert-butyl ether/ethanol.

The present disclosure further provides a method for preparing the hydrochloride crystal form II of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, the method comprising: 1) dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in a solvent (2) and adding hydrochloric acid, and 2) stirring, wherein the solvent (2) is selected from the group consisting of ethanol/ethyl acetate, methanol, ethanol/water, tetrahydrofuran/ethanol, and dichloromethane/ethanol.

The present disclosure further provides a method for preparing the maleate crystal form I of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in acetone, adding maleic acid, and stirring. The present disclosure further provides a method for preparing the fumarate crystal form I of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in tetrahydrofuran, adding fumaric acid, and stirring.

The present disclosure further provides a method for preparing the fumarate crystal form II of the compound (*S*)-3-(5-(4-((1-(4-((1*R*,2*R*)-6-hydroxy-2-isobuty,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in acetonitrile, adding fumaric acid, and stirring.

The present disclosure further provides a method for preparing the fumarate crystal form III of the compound (*S*)-3-(5-(4-((1-(4-((1*R*,2*R*)-6-hydroxy-2-isobuty,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in 1,4-dioxane, adding fumaric acid, and stirring.

The present disclosure further provides a method for preparing the fumarate crystal form IV of the compound (*S*)-3-(5-(4-((1-(4-((1*R*,2*R*)-6-hydroxy-2-isobuty,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in acetone, adding fumaric acid, and stirring.

The present disclosure further provides a method for preparing the L-tartrate crystal form I of the compound (*S*)-3-(5-(4-((1-(4-((1*R*,2*R*)-6-hydroxy-2-isobuty,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in 1,4-dioxane, adding L-tartaric acid, and stirring.

The present disclosure further provides a method for preparing the succinate crystal form I of the compound (*S*)-3-(5-(4-((1-(4-((1*R*,2*R*)-6-hydroxy-2-isobuty,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in 1,4-dioxane, adding succinic acid, and stirring.

The present disclosure further provides a method for preparing the D-malate crystal form I of the compound (*S*)-3-(5-(4-((1-(4-((1*R*,2*R*)-6-hydroxy-2-isobuty,4-tetrahydronaphthalen-1-yl)phenyl)piperidin-4-yl)methyl)piperazin-1-yl)-1-oxoisoindolin-2-yl)piperidine-2,6-dione, the method comprising the following steps: dissolving the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione in tetrahydrofuran, adding D-malic acid, and stirring.

In certain embodiments, the preparation methods for the crystal forms described in the present disclosure further comprise a crystallization, filtration, washing, or drying step.

In another aspect, the present disclosure further provides a pharmaceutical composition comprising the aforementioned crystal form A of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, or a pharmaceutically acceptable salt of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione or a crystal form thereof, and optionally a pharmaceutically acceptable excipient.

The present disclosure further provides a preparation method for a pharmaceutical composition, the method comprising a step of mixing the aforementioned crystal form A of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, or a pharmaceutically acceptable salt of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione or a crystal form thereof, with a pharmaceutically acceptable excipient.

The present disclosure further provides use of the aforementioned crystal form A of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, or a pharmaceutically acceptable salt of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione or a crystal form thereof, or the aforementioned pharmaceutical composition in the preparation of a medicament for preventing and/or treating a disorder that is treatable by degradation of a target protein that binds to a targeting ligand.

The present disclosure further provides use of the aforementioned crystal form A of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, or a pharmaceutically acceptable salt of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione or a crystal form thereof, or the aforementioned pharmaceutical composition in the preparation of a medicament for preventing and/or treating a disorder that is treatable by *in vivo* binding to cereblon.

In the present disclosure, the disorder that is treatable by degradation of a target protein that binds to a targeting ligand or the disorder that is treatable by *in vivo* binding to cereblon is selected from the group consisting of abnormal cell proliferation, tumors, immune diseases, diabetes, cardiovascular diseases, infectious diseases, and inflammatory diseases; optionally, the disease is a tumor or an infectious disease.

The tumor of the present disclosure is a cancer; optionally, the tumor is selected from the group consisting of, but is not limited to, breast cancer, endometrial cancer, uterine cancer, testicular cancer, cervical cancer, prostate cancer, ovarian cancer, fallopian tube tumors, and ovarian tumors.

The infectious disease described in the present disclosure is selected from the group consisting of, but is not limited to, viral pneumonia, influenza, avian influenza, meningitis, and gonorrhea or is an infection with HIV, HBV, HCV, HSV, HPV, RSV, CMV, Ebolavirus, Flavivirus, Pestivirus, Rotavirus, or Coronavirus.

The present disclosure further provides use of the aforementioned crystal form A of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, or a pharmaceutically acceptable salt of the compound (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione or a crystal form thereof, or the aforementioned pharmaceutical composition in the preparation of a medicament for preventing and/or treating an estrogen receptor-mediated or -dependent disease or disorder.

As used herein, "2*θ* or 2*θ* angle" refers to a diffraction angle; *θ* refers to the Bragg angle in ° or degrees; the 2*θ* of each characteristic peak has a margin of error of ±0.20 (including the case that a number having more than 1 decimal place is rounded), specifically -0.20, -0.19, -0.18, -0.17, - 0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, there is a certain degree of error in the measurement of the chemical ratio of the compound to the acid molecule. Generally, ±10% is considered a reasonable margin of error. The error varies to some extent depending on the context in which it is used, and the variation does not exceed ±10% and may be ±9%, ±8%, ±7%, ±6%, ±5%, ±4%, ±3%, ±2%, or ±1%, preferably ±5%. The numerical values described with "about" in the present disclosure have the aforementioned reasonable margin of error.

As used herein, "crystallization" or "crystallizing" includes, but is not limited to, stirring crystallization, slurrying crystallization, cooling crystallization, and volatilizing crystallization. As used herein, "differential scanning calorimetry" or "DSC" refers to the measurement of the temperature difference and heat flow difference between a sample and a reference substance during a ramping or thermostatic process to characterize all the physical changes and chemical changes related to the thermal effect, and to obtain phase change information about the sample. In the present disclosure, the drying temperature is generally 25 °C-100 °C, preferably 40 °C-70 °C, and the drying may be performed under atmospheric pressure or reduced pressure.

As used herein, "pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 shows an XRPD pattern of the amorphous form of compound I.
FIG. 2 shows an XRPD pattern of the crystal form A of compound I.
FIG. 3 shows an XRPD pattern of the hydrochloride crystal form I of compound I.
FIG. 4 shows an XRPD pattern of the hydrochloride crystal form II of compound I.
FIG. 5 shows an XRPD pattern of the maleate crystal form I of compound I.
FIG. 6 shows an XRPD pattern of the fumarate crystal form I of compound I.
FIG. 7 shows an XRPD pattern of the fumarate crystal form II of compound I.
FIG. 8 shows an XRPD pattern of the fumarate crystal form III of compound I.
FIG. 9 shows an XRPD pattern of the fumarate crystal form IV of compound I.
FIG. 10 shows an XRPD pattern of the fumarate crystal form V of compound I.
FIG. 11 shows an XRPD pattern of the L-tartrate crystal form I of compound I.
FIG. 12 shows an XRPD pattern of the succinate crystal form I of compound I.
FIG. 13 shows an XRPD pattern of the D-malate crystal form I of compound I.

### DETAILED DESCRIPTION

The present disclosure is further illustrated in detail by the following examples and experimental examples. These examples and experimental examples are illustrative only and are not intended to limit the scope of the present disclosure.

Test conditions for the instruments used in the experiments:
The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument, with dimethyl sulfoxide-D6 (DMSO-*d*₆), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q 15 Exactive).

The HPLC analyses were performed using an Agilent 1260DAD high pressure liquid chromatograph (Sunfire C18 150 × 4.6 mm chromatography column) and a Thermo U3000 high pressure liquid chromatograph (Gimini C18 150 × 4.6 mm chromatography column).

XRPD refers to X-ray powder diffraction: The analysis was performed using a BRUKER D8 X-ray diffractometer, and the specific acquisition information was: a Cu anode (40 kV, 40 mA), radiation: monochromatic Cu-Ka radiation (l = 1.5418 Å). Scan mode: *θ*/2*θ*; scan range: 3-48°. DSC refers to differential scanning calorimetry: The analysis was performed using a METTLER TOLEDO DSC 3+ differential scanning calorimeter, with a temperature ramp rate of 10 °C/min, within 25-300 °C or 25-350 °C, and a nitrogen purge speed of 50 mL/min.

TGA refers to thermogravimetric analysis: The analysis was performed using a METTLER TOLEDO TGA 2 thermogravimetric analyzer, with a temperature ramp rate of 10 °C/min, specific temperature ranges shown in corresponding patterns, and a nitrogen purge speed of 50 mL/min.

DVS refers to dynamic vapor sorption: Surface Measurement Systems instrinsic was adopted; the humidity started at 50%, the humidity range of the investigation was 0%-95%, and the humidity was increased in increments of 10%; the criterion was that each gradient mass change dM/dT is ≤ 0.002%, TMAX 360 min, two cycles.

The known starting materials in the present disclosure may be synthesized by using or following methods known in the art, or may be purchased from companies such as ABCR GmbH & Co. KG, Acros Organics, Aldrich Chemical Company, Accela ChemBio Inc., and Chembee Chemicals.

The monitoring of the reaction progress in the examples was performed by thin-layer chromatography (TLC). The developing solvent for the reactions, the eluent system for the column chromatography purification, and the developing solvent system for thin-layer chromatography include: A: a dichloromethane/methanol system, and B: a n-hexane/ethyl acetate system. The volume ratio of the solvents was adjusted depending on the polarity of the compound, or by adding a small amount of basic or acidic reagents such as triethylamine and acetic acid.

### Example 1. Preparation of Compound I (with reference to the preparation method of Example 26-1 in Application No. WO2022206737)

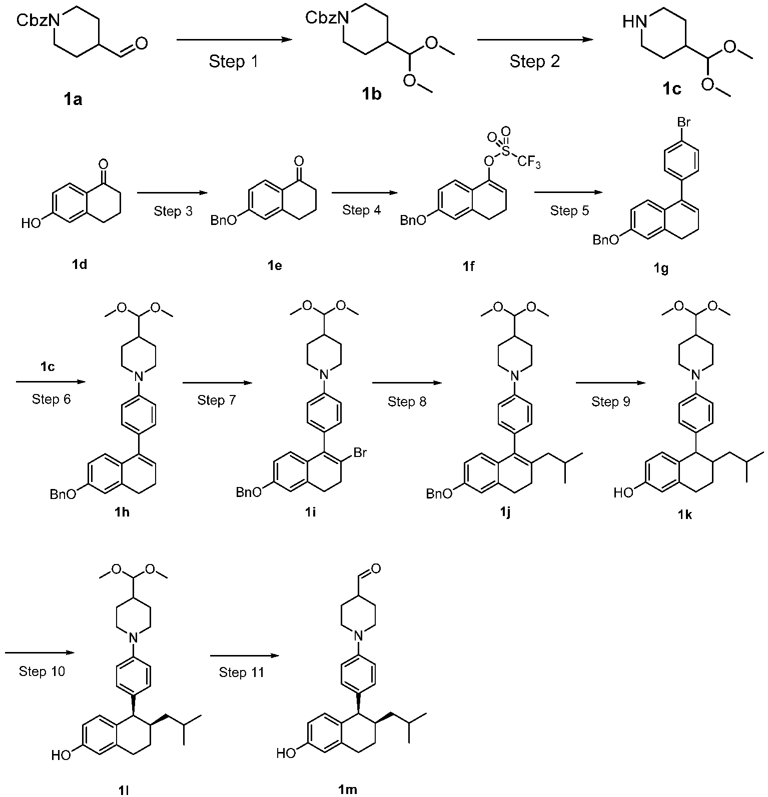

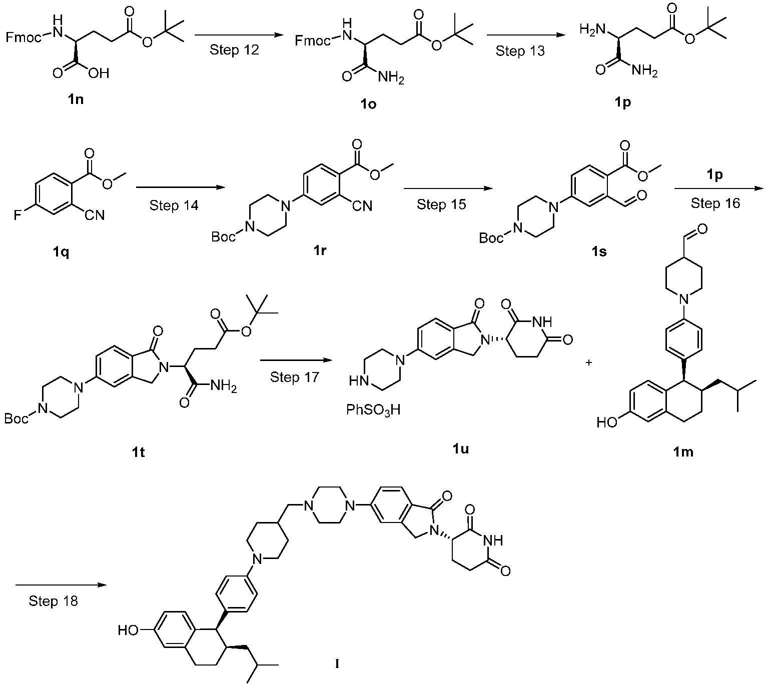

### Step 1

### Benzyl 4-(dimethoxymethyl)piperidine-1-carboxylate 1b

Benzyl 4-formylpiperidine-1-carboxylate 1a (10 g, 40.4 mmol, Shanghai Bide Pharmatech Ltd.) was dissolved in methanol (80 mL), and trimethyl orthoformate (40 mL) and p-toluenesulfonic acid monohydrate (385 mg, 2 mmol) were added. The mixture was stirred for 16 h. The reaction mixture was concentrated under reduced pressure, and a saturated sodium bicarbonate solution (80 mL) was added. Extraction was performed with ethyl acetate (80 mL × 3). The organic phases were combined, washed with a saturated sodium chloride solution (80 mL × 3), dried over anhydrous sodium sulfate, filtered, and concentrated under reduced pressure to give title compound 1b (12 g, crude). The product was directly used in the next step without purification.

### Step 2

### 4-(Dimethoxymethyl)piperidine 1c

Compound 1b (12 g, 40.9 mmol) was dissolved in methanol (100 mL), and palladium on carbon (1.3 g, 10 wt%) was added. The mixture was stirred in a hydrogen atmosphere for 3 h. The reaction mixture was filtered, and the filtrate was concentrated under reduced pressure to give title compound 1c (6 g, crude). The product was directly used in the next step without purification.

### Step 3

### 6-(Benzyloxy)-3,4-dihydronaphthalen-1(2H)-one 1e

6-Hydroxy-3,4-dihydronaphthalen-1(2H)-one 1d (8 g, 49.3 mmol, Shanghai Bide Pharmatech Ltd.) and potassium carbonate (10 g, 72.4 mmol) were added to acetonitrile (60 mL), and benzyl bromide (10 g, 58.5 mmol, 7 mL) was added dropwise. The mixture was heated at reflux for 3 h. The reaction mixture was filtered through diatomaceous earth, and the filtrate was concentrated under reduced pressure to remove the solvent. The residue was dissolved in ethyl acetate (100 mL), and the solution was washed with a saturated sodium chloride solution (20 mL × 3). The organic phase was collected, dried over anhydrous sodium sulfate, and filtered, and the filtrate was concentrated under reduced pressure to remove the solvent, giving title compound 1e (12 g, crude). The product was directly used in the next step without purification. MS m/z (ESI): 253.1[M+1].

### Step 4

### 6-(Benzyloxy)-3,4-dihydronaphthalen-1-yl trifluoromethanesulfonate 1f

Compound 1e (8 g, 31.7 mmol) was dissolved in dry tetrahydrofuran (100 mL). The reaction was cooled to -78 °C in an argon atmosphere. [Bis(trimethylsilyl)amino]lithium (1 M, 50.8 mL, 50.8 mmol) was added dropwise. After the dropwise addition, the mixture was stirred at -78 °C for 30 min. 1,1,1-Trifluoro-N-phenyl-N-(trifluoromethylsulfonyl)methanesulfonamide (17 g, 47.6 mmol) was slowly added. The mixture was naturally warmed to room temperature and stirred for 2 h. Water (100 mL) was slowly added to quench the reaction, and extraction was performed with dichloromethane (50 mL × 3). The organic phases were combined, dried over anhydrous sodium sulfate, and filtered, and the filtrate was concentrated under reduced pressure to remove the solvent. The residue was purified by silica gel column chromatography using eluent system B to give title compound 1f (10.1 g, yield: 83%). MS m/z (ESI): 385.2[M+1].

### Step 5

### 7-(Benzyloxy)-4-(4-bromophenyl)-1,2-dihydronaphthalene 1g

Compound 1f (32 g, 83.3 mmol), 4-bromobenzeneboronic acid (20 g, 100 mmol, Shanghai Meryer Biochemical Technology Co., Ltd.), tetrakis(triphenylphosphine)palladium(0) (9.62 g, 8.3 mmol), and sodium carbonate (26.47 g, 250 mmol) were sequentially added to 360 mL of a mixed solvent of 1,4-dioxane and water (V/V = 5/1). The mixture was left to react at 80 °C for 2 h in a nitrogen atmosphere. The reaction mixture was cooled to room temperature. Water (200 mL) was added, and extraction was performed with dichloromethane (200 mL × 3). The organic phases were combined, washed with a saturated sodium chloride solution (200 mL × 2), dried over anhydrous sodium sulfate, and filtered, and the filtrate was concentrated under reduced pressure to remove the solvent. The residue was purified by silica gel column chromatography using eluent system B to give title compound 1g (14 g, yield: 43%).

### Step 6

### 1-(4-(6-(Benzyloxy)-3,4-dihydronaphthalen-1-yl)phenyl)-4-(dimethoxymethyl)piperidine 1h

Compound 1g (16 g, 40.9 mmol), compound 1c (7.81 g, 49.1 mmol), palladium acetate (1.38 g, 6.1 mmol), 2-dicyclohexylphosphino-2',4',6'-triisopropylbiphenyl (3.9 g, 8.2 mmol), and sodium tert-butoxide (11.79 g, 123 mmol) were added to toluene (350 mL). The mixture was left to react at 90 °C for 2 h in a nitrogen atmosphere. The reaction mixture was cooled to room temperature. Water (100 mL) was added, and extraction was performed with dichloromethane (200 mL × 3). The organic phases were combined, washed with a saturated sodium chloride solution (100 mL × 2), dried over anhydrous sodium sulfate, and filtered, and the filtrate was concentrated under reduced pressure to remove the solvent. The residue was purified by silica gel column chromatography using eluent system A to give title compound 1h (10.5 g, yield: 55%). MS m/z (ESI): 470.2[M+1].

### Step 7

### 1-(4-(6-(Benzyloxy)-2-bromo-3,4-dihydronaphthalen-1-yl)phenyl)-4-(dimethoxymethyl)piperidine 1i

Compound 1h (10.5 g, 22.4 mmol) was dissolved in dichloromethane (350 mL), and the solution was cooled to -5 °C in an ice-salt bath. Pyridinium tribromide (8.58 g, 26.8 mmol) and triethylamine (4.52 g, 44.7 mmol) were added portionwise. The mixture was left to react at -5 °C for 30 min. A saturated sodium bicarbonate solution (100 mL) was added to the reaction mixture. The organic phase was separated, washed with a saturated sodium chloride solution (100 mL × 3), dried over anhydrous sodium sulfate, and filtered, and the filtrate was concentrated under reduced pressure to remove the solvent. The residue was purified by silica gel column chromatography using eluent system B to give title product 1i (5.1 g, yield: 42%). MS m/z (ESI): 550.2[M+1].

### Step 8

### 1-(4-(6-(Benzyloxy)-2-isobutyl-3,4-dihydronaphthalen-1-yl)phenyl)-4-(dimethoxymethyl)piperidine 1j

In a nitrogen atmosphere, a solution of zinc chloride in tetrahydrofuran (1 M, 8.2 mL) was slowly added dropwise to an ice-bath-cooled solution of tert-butylmagnesium chloride in tetrahydrofuran (1 M, 7.5 mL, Shanghai Adamas Co., Ltd.). After the dropwise addition, the mixture was left to react at room temperature for 3 h. A solution of compound 1i (400 mg, 0.73 mmol) and methanesulfonato(2-dicyclohexylphosphino-2',6'-diisopropoxy-1,1'-biphenyl)(2-amino-1,1'-biphenyl-2-yl)palladium(II) (Ruphos-Pd G3, 90 mg, 0.11 mmol, Jiangsu Aikon Biopharmaceutical R&D Co., Ltd.) in tetrahydrofuran (2 mL) was added. After the dropwise addition, the mixture was left to react at room temperature for 16 h. A saturated ammonium chloride solution (10 mL) was added, and the organic phase was separated. The aqueous phase was extracted with dichloromethane (15 mL × 2). The organic phases were combined, dried over anhydrous sodium sulfate, filtered, and concentrated under reduced pressure to remove the solvent, and the residue was purified by silica gel column chromatography using eluent system B to give title compound 1j (300 mg, yield: 75%). MS m/z (ESI): 526.3[M+1].

### Step 9

### 5-(4-(4-(Dimethoxymethyl)piperidin-1-yl)phenyl)-6-isobutyl-5,6,7,8-tetrahydronaphthalen-2-ol1k

Compound 1j (130 mg, 0.25 mmol) was dissolved in methanol (10 mL), and palladium hydroxide on carbon (100 mg, 20 wt%) was added. The mixture was left to react at room temperature for 16 h in a hydrogen atmosphere. The reaction mixture was filtered, and the filtrate was concentrated under reduced pressure to remove the solvent, giving title compound 1k (90 mg, yield: 83%). MS m/z (ESI): 438.3[M+1].

### Step 10

### (5R,6R)-5-(4-(4-(Dimethoxymethyl)piperidin-1-yl)phenyl)-6-isobutyl-5,6,7,8-tetrahydronaphthalen-2-ol 1l

Compound 1k (90 mg, 0.21 mmol) was resolved by preparative chiral chromatography (resolution conditions: chromatography column: CHIRALPAK IE, 20 mm × 250 mm, 5 µm; mobile phases: A: n-hexane, B: ethanol (+ 20 mmol NH₃), A: 85%, B: 15%, flow rate: 20 mL/min) to give title compound 1l (31 mg). MS m/z (ESI): 438.3[M+1].

### Step 11

### 1-(4-((1R,2R)-6-Hydroxy-2-isobutyl-1,2,3,4-tetrahydronaphthalen-1-yl)phenyl)piperidine-4-carbaldehyde 1m

Compound 1l (35 mg, 0.08 mmol) was dissolved in tetrahydrofuran (2.5 mL), and dilute sulfuric acid (2 M, 0.15 mL, 0.3 mmol) was added. The mixture was heated to 55 °C and left to react for 1 h. The reaction mixture was cooled to room temperature, made neutral with a saturated sodium bicarbonate solution, and extracted with ethyl acetate (10 mL × 3). The organic phases were combined, dried over anhydrous sodium sulfate, and filtered. The reaction mixture was concentrated under reduced pressure to remove the solvent, giving title compound 1m (31 mg, yield: 99%). MS m/z (ESI): 392.2[M+1].

### Step 12

### tert-Butyl (S)-4-((((9H-fluoren-9-yl)methoxy)carbonyl)amino)-5-amino-5-oxopentanoate 1o

(*S*)-2-((((9H-Fluoren-9-yl)methoxy)carbonyl)amino)-5-(tert-butoxy)-5-oxopentanoic acid 1n (40 g, 94 mmol, Shanghai Hanhong Scientific Co., Ltd.) and di-tert-butyl dicarbonate (32.83 g, 150 mmol) were added to 1,4-dioxane (300 mL). The internal temperature was controlled at below 5 °C using an ice-water bath in a nitrogen atmosphere, and pyridine (15 mL, 188 mmol) was added dropwise. After the dropwise addition, the mixture was left to react in an ice-water bath for 0.5 h. Ammonium bicarbonate (66.89 g, 282 mmol) was added. The mixture was warmed to room temperature and left to react for 12 h. The reaction mixture was concentrated under reduced pressure to remove the solvent, and ethyl acetate (500 mL) was added. The mixture was washed with dilute hydrochloric acid (500 mL × 3) and filtered. The filtrate was concentrated under reduced pressure to remove the solvent, giving title compound 1o (45.3 g, crude). The product was directly used in the next step without purification. MS m/z (ESI): 369.1[M-55].

### Step 13

### tert-Butyl (S)-4,5-diamino-5-oxopentanoate 1p

Compound 1o (45.3 g, 94 mmol) and diethylamine (50 mL) were added to dichloromethane (500 mL). The mixture was left to react at room temperature for 12 h. The reaction mixture was concentrated under reduced pressure to remove the solvent. The residue was dissolved in methanol (150 mL), and water (5 mL) was added. The mixture was washed with n-heptane (150 mL × 3). The methanol layer was concentrated under reduced pressure to remove the solvent, giving title compound 1p (21.5 g, crude). The product was directly used in the next step without purification. MS m/z (ESI): 203.1[M+1].

### Step 14

### tert-Butyl 4-(3-cyano-4-(methoxycarbonyl)phenyl)piperazine-1-carboxylate 1r

Methyl 2-cyano-4-fluorobenzoate 1q (50 g, 0.28 mol, Jiangsu Aikon Biopharmaceutical R&D Co., Ltd.), tert-butyl piperazine-1-carboxylate acetate (62.3 g, 0.34 mol), and isopropylethylamine (250 mL, 1.39 mol) were added to tetrahydrofuran (1 L). The mixture was left to react at 120 °C for 12 h. Water (1 L) was added to the reaction mixture, and extraction was performed with ethyl acetate (1 L × 3). The organic phases were combined, washed with a saturated sodium chloride solution (1 L × 2), dried over anhydrous sodium sulfate, and filtered, and the filtrate was concentrated under reduced pressure to remove the solvent, giving title compound 1r (89 g, crude). The product was directly used in the next step without purification. MS m/z (ESI): 290.1[M-55].

### Step 15

### tert-Butyl 4-(3-formyl-4-(methoxycarbonyl)phenyl)piperazine-1-carboxylate 1s

Compound 1r (5 g, 14.5 mmol), pyridine (10.5 mL), glacial acetic acid (6.6 mL), and Raney nickel (2.5 g) were added to water (5 mL), and the temperature was raised to 70 °C. Sodium hypophosphite (7.5 g) was dissolved in water (15 mL), and the solution was added dropwise to the reaction mixture. After the dropwise addition, the mixture was left to react at 70 °C for 12 h. The reaction mixture was cooled to room temperature, and ethyl acetate (50 mL) and water (50 mL) were added. The organic phase was separated, washed with dilute hydrochloric acid (1 M, 50 mL × 3), washed with a saturated sodium chloride solution (50 mL × 3), dried over anhydrous sodium sulfate, and filtered, and the filtrate was concentrated under reduced pressure to remove the solvent. The residue was purified by silica gel column chromatography using eluent system A to give title compound 1s (3 g, yield: 59%). MS m/z (ESI): 293.1[M-55].

### Step 16

### tert-Butyl (S)-4-(2-(1-amino-5-(tert-butoxy)-1,5-dioxopentan-2-yl)-1-oxoisoindolin-5-yl)piperazine-1-carboxylate 1t

Compound 1s (1.3 g, 3.7 mmol) and compound 1p (0.89 g, 4.5 mmol) were added to methanol (10 mL). The internal temperature was controlled at below 5 °C using an ice-water bath, and acetic acid (0.3 mL, 5.6 mmol) and sodium cyanoborohydride (0.46 g, 7.46 mmol) were added dropwise. The mixture was left to react at room temperature for 12 h. The reaction mixture was concentrated under reduced pressure to remove the solvent, and ethyl acetate (50 mL) and water (50 mL) were added to the residue. The organic phase was separated, washed with a saturated citric acid solution (50 mL × 3), dried over anhydrous sodium sulfate, and filtered. The filtrate was concentrated under reduced pressure to remove the solvent, and the residue was purified by silica gel column chromatography using eluent system A to give title product 1t (0.71 g, yield: 38%). MS m/z (ESI): 503.2[M+1].

### Step 17

### (S)-3-(1-Oxo-5-(piperazin-1-yl)isoindolin-2-yl)piperidine-2,6-dione benzenesulfonate 1u

Compound 1t (5.7 g, 11.4 mmol) and benzenesulfonic acid (3.59 g, 22.7 mmol) were added to acetonitrile (15 mL), and the mixture was stirred at 90 °C for 12 h. The reaction mixture was concentrated under reduced pressure to remove the solvent, and the residue was washed with ethyl acetate (100 mL × 3) and dried to give title compound 1u (5.7 g, yield: 100%). MS m/z (ESI): 329.1[M+1].

### Step 18

### (S)-3-(5-(4-((1-(4-((1R,2R)-6-Hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione 1

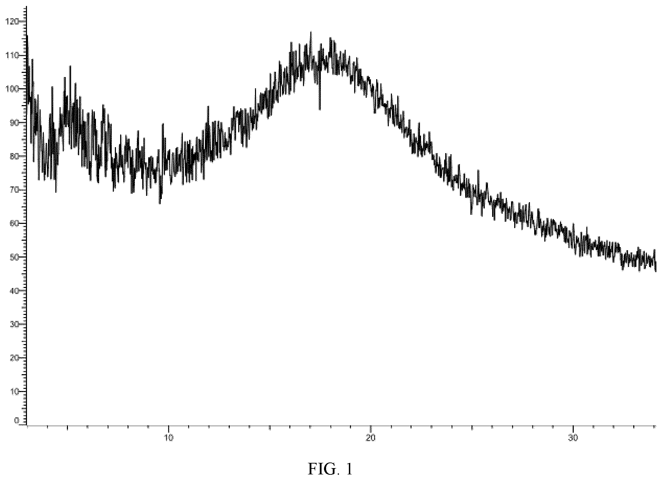

Compound 1u (50 mg, 0.1 mmol) was added to a mixed solvent of dichloromethane and methanol (V/V = 4/1, 5 mL), and sodium acetate (130 mg, 1.58 mmol) was added. The mixture was left to react for 10 min, and compound 1m (31 mg, 0.08 mmol) was added. The mixture was left to react for 15 min, and sodium triacetoxyborohydride (34 mg, 0.16 mmol) was added. The mixture was left to react at room temperature for 1 h. The reaction mixture was concentrated under reduced pressure to remove the solvent, and the residue was purified by preparative high performance liquid chromatography (Waters 2767-SQ Detecor2, elution system: ammonium bicarbonate: 10 mM, water: 60%, acetonitrile: 40%) to give compound 1 (25 mg, yield: 45%). MS m/z (ESI): 704.4[M+1]. According to an X-ray powder diffraction analysis, the product was an amorphous form. The X-ray powder diffraction pattern of the amorphous form is shown in FIG. 1.

### Example 2. Inhibitory Effect of Compound I on MCF7 Cell Proliferation

MCF7 cells (TCHu74, National Collection of Authenticated Cell Cultures) were cultured in an MEM (GE Healthcare, SH30024.01) complete culture medium containing 10% fetal bovine serum. On day 1 of the experiment, MCF7 cells were seeded into a 96-well plate at a density of 3,000 cells/well using an MEM culture medium containing 2% fetal bovine serum, with each well containing 135 µL of cell suspension. The plate was incubated overnight in a 37 °C, 5% CO₂ cell incubator. The next day, the test compound, at different concentrations in the culture medium, was added at 15 µL/well. The final concentrations of the compound were 9 concentration points obtained by 4-fold serial dilution from 1000 nM. A blank control containing 0.5% DMSO was set up. The plate was incubated in a 37 °C, 5% CO₂ cell incubator for 6 days. On day 8, the 96-well cell culture plate was taken out, and CellTiter-Glo^{®} Luminescent Cell Viability Assay (Promega, G7573) was added at 75 µL/well. After the plate was left at room temperature for 10 min, luminescence signal values were measured using a multilabel microplate reader (PerkinElmer, VICTOR 3). According to a calculation based on the concentrations of the compound and the luminescence signal values using Graphpad Prism software, the IC50 value for the inhibitory activity of the compound is 0.59 nM.

### Example 3. Pharmacodynamic Study of Compound I

### 1. Objective

To evaluate the inhibitory effect of compound I on the growth of xenograft tumors of MCF-7 (Y537S) human breast cancer cells in BEIGE SCID mice.

### 2. Test compound

Compound I. A 2% Tween 80 + 98% PEG-400 solution was used.

### 3. Method and materials

### 3.1. Test animals and housing conditions

Test animals: female BEIGE SCID mice, purchased from Beijing Vital River Laboratory Animal Technology Co., Ltd. (certificate No. SCXK (Beijing) 2016-0006), weighing about 19 g on purchase.

Housing conditions: 5 animals/cage, a 12/12-hour light/dark cycle, a constant temperature of 23 ± 1 °C with humidity at 50 to 60%, *ad libitum* access to food and water.

### 3.2. Grouping of animals

After acclimatization, BEIGE SCID mice were grouped as follows:

| Group | Number of animals | Mode of administration |
|---|---|---|
| Vehicle control | 8 | 2% Tween 80 + 98% PEG-400 solution (i.g./qd) |
| Compound I | 8 | 5 mpk (i.g./qd) |
| Compound I | 8 | 15 mpk (i.g./qd) |
| Compound I | 8 | 45 mpk (i.g./qd) |

| | | |
|---|---|---|
| Notes: qd stands for "once a day"; i.g. stands for intragastric administration. | | |

### 3.3. Method:

MCF-7 (Y537S) cells in the logarithmic growth phase were subcutaneously inoculated into the right flank of female BEIGE SCID mice at 1.0 × 10⁷/mouse/200 µL (containing 100 µL of matrigel). After 18 days, when the tumor volume of tumor-bearing mice reached about 170 mm³, the mice were randomly divided into 4 groups of 8 according to tumor volume and body weight: a vehicle control group, a compound I 5 mpk group, a compound I 15 mpk group, and a compound I 45 mpk group. The day of grouping was set as D₀, and intragastric administration was started and performed once a day for 28 days. The 28th day post-dose was set as D₂₈ (Table 1). The tumor volume of tumor-bearing mice was measured with a vernier caliper twice a week.

### 3.4. Statistical analysis

All data were plotted and statistically analyzed using Excel and GraphPad Prism 8 software. The calculation formula for tumor volume (V) was: V = 1/2 × a × b2, where a and b represent length and width, respectively.

Relative tumor proliferation rate T/C (%) = (T - T₀)/(C - C₀) × 100 (%), where T and C represent the tumor volumes of the treatment groups and the control group at the end of the experiment; T₀ and C₀ represent the tumor volumes at the beginning of the experiment.

Tumor growth inhibition rate TGI (%) = 1 - T/C (%). If TGI (%) exceeds 100%, its specific value will not be shown; instead, it will be represented as >100%. Tumor regression (%) = [(T₀ - T)/T₀] × 100 (%).

### 4. Results

Data on the efficacy of compound I against MCF-7 (Y537S) xenograft tumors in BEIGE SCID mice are shown in Table 1.

**Table 1. The efficacy of compound I against MCF-7 (Y537S) xenograft tumors in BEIGE SCID mice**

| Group | Administration | Route Dose (mpk) | Mean tumor volume (mm³) | | | | TGI (%) D₂₈ | p | Number of remaining animals/ group |
|---|---|---|---|---|---|---|---|---|---|
| | | | D₀ | SEM | D₂₈ | SEM | | (vs. vehicle control) | |
| Vehicle control | qd/28d | i.g., 0 | 171.7 | 11.8 | 657.3 | 73.4 | / | / | 8/8 |
| Compound I | qd/28d | i.g., 5 | 170.2 | 11.8 | 305.0 | 23.7 | 72 | <0.001 | 8/8 |
| Compound I | qd/28d | i.g., 15 | 168.2 | 12.8 | 219.7 | 15.7 | 89 | <0.001 | 8/8 |
| Compound I | qd/28d | i.g., 45 | 168.6 | 13.0 | 192.0 | 12.3 | 95 | <0.001 | 8/8 |

| | | | | | | | | | |
|---|---|---|---|---|---|---|---|---|---|
| Notes: qd stands for "once a day"; d stands for day; i.g. stands for intragastric administration; SEM stands for standard error of measurement. | | | | | | | | | |

### 5. Conclusion

The administration of compound I was started and performed once a day 18 days after tumor cell grafting. Twenty-eight days after the administration, the 5 mpk low-dose group showed a tumor growth inhibition rate of 72%, the 15 mpk medium-dose group showed a tumor growth inhibition rate of 89%, and the 45 mpk high-dose group showed a tumor growth inhibition rate of 95%.

### Example 4. Preparation of Amorphous Form of Compound

5 mg of compound I was weighed out and dissolved in a solvent to give a product. According to an X-ray powder diffraction analysis, no significant characteristic peaks were observed, which indicates that the product was an amorphous form. The XRPD pattern of the amorphous form is shown in FIG. 1.

**Table 2. The preparation of the amorphous form of the compound**

| | Solvent | Procedure | Crystal form |
|---|---|---|---|
| 1 | 0.5 mL of isopropanol | Slurry for 3 days | Amorphous form |
| 2 | 0.5 mL of water | | Amorphous form |
| 3 | 0.5 mL of isopropyl acetate | | Amorphous form |
| 4 | 0.5 mL of methyl tert-butyl ether | | Amorphous form |
| 5 | 0.5 mL of n-heptane | | Amorphous form |
| 6 | 0.5 mL of nitromethane | | Amorphous form |
| 7 | 0.5 mL of 50% methanol/water | | Amorphous form |
| 8 | 0.5 mL of ethyl acetate/n-heptane | | Amorphous form |
| 9 | 0.5 mL of cyclohexane | | Amorphous form |
| 10 | 0.5 mL of n-hexane | | Amorphous form |
| 11 | 0.5 mL of isopropyl ether | | Amorphous form |
| 12 | 0.5 mL of toluene | | Amorphous form |
| 13 | 0.05 mL of acetone | Add 0.2 mL of methyl tert-butyl ether | Amorphous form |
| 14 | 0.05 mL of 2-butanone | | Amorphous form |
| 15 | 0.05 mL of tetrahydrofuran | | Amorphous form |
| 16 | 0.05 mL of dichloromethane | | Amorphous form |
| 17 | 0.05 mL of 50% acetonitrile/methanol | | Amorphous form |
| 18 | 0.05 mL of methanol/chloroform | | Amorphous form |
| 19 | 0.05 mL of trichloromethane | | Amorphous form |
| 20 | 0.05 mL of 1,4-dioxane | Add 0.45 mL of methyl tert-butyl ether | Amorphous form |
| 21 | 0.05 mL of propylene glycol methyl ether | | Amorphous form |
| 22 | 0.05 mL of 10% water/acetone | | Amorphous form |
| 23 | 0.05 mL of 50% ethyl acetate/ethanol | | Amorphous form |
| 24 | 0.05 mL of tetrahydrofuran/ethanol (2:1) | | Amorphous form |
| 25 | 0.05 mL of dimethyl sulfoxide | Add 0.2 mL of water | Amorphous form |
| 26 | 0.05 mL of N-methylpyrrolidone | | Amorphous form |
| 27 | 0.05 mL of N,N-dimethylformamide | | Amorphous form |
| 28 | 0.05 mL of N,N-dimethylacetamide | | Amorphous form |

### Example 5. Preparation of Crystal Form A of Compound

About 24 mg of compound I was weighed out and dissolved in 0.7 mL of 50% ethanol/ethyl acetate, and a hydrochloric acid solution (2 mol/L, 37 µL) was added. The reaction mixture was slurried for 1 day and centrifuged, and a 0.2 M phosphate buffer was added to the solid to adjust the pH to 6. After 1 day of slurrying, the solid was centrifuged and dried to give a product. According to an X-ray powder diffraction analysis, the product was defined as crystal form A of the compound. The XRPD pattern of the crystal form is shown in FIG. 2, and its characteristic peak positions are shown in Table 3. A TGA profile of the crystal form shows a weight loss of 2.9% before 140 °C. A DSC profile of the crystal form shows an endothermic peak at 162.58 °C.

**Table 3. The peak positions of crystal form A of the compound**

| Peak No. | 2*θ* value [° or degrees] | d[Å] | Relative intensity% |
|---|---|---|---|
| 1 | 5.024 | 17.57618 | 41.5 |
| 2 | 5.998 | 14.72287 | 33.0 |
| 3 | 14.232 | 6.21805 | 95.1 |
| 4 | 15.321 | 5.77861 | 83.8 |
| 5 | 16.088 | 5.50479 | 60.6 |
| 6 | 17.430 | 5.08394 | 54.0 |
| 7 | 19.102 | 4.64249 | 100.0 |
| 8 | 19.977 | 4.44099 | 54.6 |
| 9 | 20.938 | 4.23934 | 48.7 |
| 10 | 22.317 | 3.98039 | 84.9 |
| 11 | 24.809 | 3.58596 | 21.3 |
| 12 | 26.882 | 3.31395 | 19.4 |
| 13 | 27.526 | 3.23788 | 15.1 |
| 14 | 28.903 | 3.08667 | 19.0 |
| 15 | 30.093 | 2.96718 | 19.4 |

### Example 6. Preparation of Hydrochloride Crystal Form I of Compound

48 mg of compound I was weighed out and dissolved in 1.4 mL of 50% ethanol/methyl tert-butyl ether, and a hydrochloric acid solution (2 mol/L, 74 µL) was added. The reaction mixture was slurried for 1 day and centrifuged, and drying was performed to give a product.

According to an X-ray powder diffraction analysis, the product was defined as hydrochloride crystal form I. The XRPD pattern of the crystal form is shown in FIG. 3, and its characteristic peak positions are shown in Table 4. According to an ion chromatography analysis, the chloride ion content was 9.08%. A DSC profile of the crystal form shows endothermic peaks at 72.79 °C and 248.5 °C. A TGA profile of the crystal form shows a weight loss of 2.8% from 30 °C to 120 °C.

DVS tests showed that the sample had a hygroscopic weight gain of about 6.3% under normal storage conditions (i.e., 25 °C and 60% RH), a hygroscopic weight gain of about 7.3% under accelerated experimental conditions (i.e., 70% RH), and a hygroscopic weight gain of about 8.9% under extreme conditions (i.e., 90% RH). After the DVS tests, the sample was re-identified, and the results showed that its crystal form did not change.

**Table 4. The peak positions of hydrochloride crystal form I**

| Peak No. | 2*θ* value [° or degrees] | d[Å] | Relative intensity% |
|---|---|---|---|
| 1 | 5.597 | 15.77743 | 4.9 |
| 2 | 6.116 | 14.43876 | 4.0 |
| 3 | 10.784 | 8.19726 | 8.7 |
| 4 | 13.751 | 6.43442 | 100.0 |
| 5 | 15.084 | 5.86870 | 2.5 |
| 6 | 15.883 | 5.57547 | 15.4 |
| 7 | 16.917 | 5.23668 | 12.5 |
| 8 | 17.881 | 4.95669 | 9.7 |
| 9 | 18.981 | 4.67185 | 75.1 |
| 10 | 20.097 | 4.41475 | 38.5 |
| 11 | 20.592 | 4.30979 | 9.8 |
| 12 | 22.816 | 3.89449 | 34.3 |
| 13 | 25.168 | 3.53560 | 14.5 |
| 14 | 26.460 | 3.36585 | 14.1 |
| 15 | 27.169 | 3.27960 | 3.5 |
| 16 | 28.207 | 3.16114 | 5.0 |
| 17 | 30.815 | 2.89930 | 13.0 |

### Example 7. Preparation of Hydrochloride Crystal Form I of Compound

48 mg of compound I was weighed out, a solvent and a 2 mol/L hydrochloric acid solution were added, and a product was obtained. According to an X-ray powder diffraction analysis, the product was hydrochloride crystal form I.

**Table 5. The preparation of hydrochloride crystal form I of the compound**

| | Solvent | Procedure | Crystal form |
|---|---|---|---|
| 1 | 1.4 mL of methanol | Add a hydrochloric acid solution (2 mol/L, 74 µL), slurry the reaction mixture for 1 day, and perform centrifugation and drying | Hydrochloride crystal form I |
| 2 | 1.4 mL of 93% ethanol/water | | Hydrochloride crystal form I |
| 3 | 1.4 mL of 67% tetrahydrofuran/ethanol | | Hydrochloride crystal form I |
| 4 | 1.4 mL of 50% dichloromethane/ethanol | | Hydrochloride crystal form I |
| 5 | 1.4 mL of 50% ethyl acetate/ethanol | | Hydrochloride crystal form I |

### Example 8. Preparation of Hydrochloride Crystal Form I of Compound

Hydrochloride crystal form II was heated to 120 °C to give a product. According to an X-ray powder diffraction analysis, the product was hydrochloride crystal form I.

### Example 9. Preparation of Hydrochloride Crystal Form II of Compound

About 48 mg of compound I was weighed out and dissolved in 1.4 mL of 50% ethanol/ethyl acetate, and a hydrochloric acid solution (2 mol/L, 74 µL) was added. The reaction mixture was slurried for 1 day and centrifuged to give a product.

According to an X-ray powder diffraction analysis, the product was defined as hydrochloride crystal form II. The XRPD pattern of the crystal form is shown in FIG. 4, and its characteristic peak positions are shown in Table 6. According to an ion chromatography analysis, the chloride ion content was 8.72%. A DSC profile of the crystal form shows endothermic peaks at 66.12 °C, 120.27 °C, and 248.83 °C. A TGA profile of the crystal form shows a weight loss of 1.9% from 30 °C to 160 °C.

**Table 6. The peak positions of hydrochloride crystal form II**

| Peak No. | 2*θ* value [° or degrees] | d[Å] | Relative intensity% |
|---|---|---|---|
| 1 | 5.557 | 15.89031 | 14.3 |
| 2 | 8.770 | 10.07489 | 9.4 |
| 3 | 10.406 | 8.49444 | 19.8 |
| 4 | 13.184 | 6.70994 | 64.2 |
| 5 | 13.763 | 6.42881 | 21.4 |
| 6 | 14.452 | 6.12382 | 19.9 |
| 7 | 15.071 | 5.87372 | 25.9 |
| 8 | 17.072 | 5.18956 | 100.0 |
| 9 | 18.215 | 4.86648 | 10.9 |
| 10 | 19.673 | 4.50900 | 38.6 |
| 11 | 20.564 | 4.31552 | 67.8 |
| 12 | 22.786 | 3.89951 | 71.1 |
| 13 | 23.326 | 3.81046 | 17.6 |
| 14 | 24.211 | 3.67312 | 4.0 |
| 15 | 24.971 | 3.56306 | 40.5 |
| 16 | 26.102 | 3.41113 | 25.9 |
| 17 | 26.571 | 3.35194 | 13.5 |
| 18 | 27.674 | 3.22089 | 11.4 |
| 19 | 28.604 | 3.11820 | 10.6 |
| 20 | 29.588 | 3.01675 | 13.9 |
| 21 | 29.883 | 2.98763 | 11.8 |
| 22 | 30.735 | 2.90669 | 4.8 |
| 23 | 32.571 | 2.74692 | 9.2 |
| 24 | 33.620 | 2.66357 | 9.6 |

### Example 10. Preparation of Hydrochloride Crystal Form II of Compound

48 mg of compound I was weighed out, a solvent and a 2 mol/L hydrochloric acid solution were added, and a product was obtained. According to an X-ray powder diffraction analysis, the product was hydrochloride crystal form II.

**Table 7. The preparation of hydrochloride crystal form II of the compound**

| | Solvent | Procedure | Crystal form |
|---|---|---|---|
| 1 | 1.4 mL of methanol | Add a hydrochloric acid solution (2 mol/L, 74 µL), slurry the reaction mixture overnight, and perform centrifugation | Hydrochloride crystal form II |
| 2 | 1.4 mL of 93% ethanol/water | | Hydrochloride crystal form II |
| 3 | 1.4 mL of 67% tetrahydrofuran/ethanol | | Hydrochloride crystal form II |
| 4 | 1.4 mL of 50% dichloromethane/ethanol | | Hydrochloride crystal form II |

### Example 11. Preparation of Amorphous Hydrochloride of Compound

About 15 mg of compound I was weighed out and dissolved in 0.2 mL of 50% ethanol/ethyl acetate, and a hydrochloric acid solution (2 mol/L, 11 µL) was added. The reaction mixture was slurried for 1 day and centrifuged, and drying was performed to give a product.

According to an X-ray powder diffraction analysis, no significant characteristic peaks were observed, which indicates that the product was an amorphous hydrochloride. According to an ion chromatography analysis, the chloride ion content was 5.66%.

### Example 12. Preparation of Maleate Crystal Form I of Compound

About 100 mg of compound I was weighed out and dissolved in 3 mL of acetone, and 18.2 mg of maleic acid was added. The reaction mixture was slurried for 1 day to give a product.

According to an X-ray powder diffraction analysis, the product was defined as maleate crystal form I. The XRPD pattern of the crystal form is shown in FIG. 5, and its characteristic peak positions are shown in Table 8. According to an ion chromatography analysis, the maleate ion content was 12.53%. A DSC profile of the crystal form shows an endothermic peak at 145.8 °C. A TGA profile of the crystal form shows a weight loss of 1% from 30 °C to 90 °C and a weight loss of 12.4% from 90 °C to 190 °C.

**Table 8. The peak positions of maleate crystal form I**

| Peak No. | 2*θ* value [° or degrees] | d[Å] | Relative intensity% |
|---|---|---|---|
| 1 | 5.626 | 15.69616 | 59.9 |
| 2 | 7.017 | 12.58699 | 12.8 |
| 3 | 8.373 | 10.55160 | 15.5 |
| 4 | 8.820 | 10.01748 | 43.7 |
| 5 | 9.386 | 9.41467 | 100.0 |
| 6 | 10.200 | 8.66542 | 64.5 |
| 7 | 10.683 | 8.27429 | 50.8 |
| 8 | 11.259 | 7.85241 | 16.4 |
| 9 | 13.209 | 6.69758 | 10.9 |
| 10 | 14.966 | 5.91481 | 6.6 |
| 11 | 15.888 | 5.57362 | 39.3 |
| 12 | 16.777 | 5.28018 | 15.0 |
| 13 | 17.534 | 5.05400 | 36.0 |
| 14 | 17.953 | 4.93690 | 59.0 |
| 15 | 18.230 | 4.86250 | 47.2 |
| 16 | 20.048 | 4.42557 | 17.0 |
| 17 | 20.586 | 4.31102 | 27.9 |
| 18 | 21.514 | 4.12714 | 17.0 |
| 19 | 22.266 | 3.98942 | 22.6 |
| 20 | 22.834 | 3.89140 | 11.8 |
| 21 | 23.916 | 3.71776 | 18.0 |
| 22 | 25.433 | 3.49937 | 12.7 |
| 23 | 26.703 | 3.33577 | 11.8 |
| 24 | 26.965 | 3.30392 | 11.8 |
| 25 | 31.063 | 2.87676 | 10.1 |
| 26 | 32.571 | 2.74692 | 8.4 |

### Example 13. Preparation of Fumarate Crystal Form I of Compound

About 98 mg of compound I was weighed out and dissolved in 3 mL of tetrahydrofuran, and 17.1 mg of fumaric acid was added. The reaction mixture was slurried for 1 day and centrifuged, and drying was performed to give the title product.

According to an X-ray powder diffraction analysis, the product was defined as fumarate crystal form I. The XRPD pattern of the crystal form is shown in FIG. 6, and its characteristic peak positions are shown in Table 9. According to an ion chromatography analysis, the fumarate ion content was 11.58%. A DSC profile of the crystal form shows an endothermic peak at 141.44 °C. A TGA profile of the crystal form shows a weight loss of 13.6% from 30 °C to 160 °C.

**Table 9. The peak positions of fumarate crystal form I**

| Peak No. | 2*θ* value [° or degrees] | d[Å] | Relative intensity% |
|---|---|---|---|
| 1 | 5.661 | 15.59773 | 37.1 |
| 2 | 6.803 | 12.98294 | 38.6 |
| 3 | 8.934 | 9.89040 | 17.7 |
| 4 | 9.590 | 9.21558 | 54.6 |
| 5 | 10.450 | 8.45899 | 100.0 |
| 6 | 11.261 | 7.85084 | 23.9 |
| 7 | 16.015 | 5.52964 | 31.0 |
| 8 | 17.557 | 5.04729 | 37.8 |
| 9 | 18.238 | 4.86032 | 40.2 |
| 10 | 19.799 | 4.48066 | 37.1 |
| 11 | 21.117 | 4.20380 | 43.3 |
| 12 | 22.178 | 4.00494 | 20.0 |
| 13 | 22.834 | 3.89140 | 13.2 |
| 14 | 23.752 | 3.74305 | 1.6 |
| 15 | 24.998 | 3.55926 | 12.4 |

### Example 14. Preparation of Fumarate Crystal Form II of Compound

About 7.1 mg of compound I was weighed out and dissolved in 0.2 mL of acetonitrile, and 2.3 mg of fumaric acid was added. The reaction mixture was slurried for 1 day and centrifuged, and drying was performed to give a product.

According to an X-ray powder diffraction analysis, the product was defined as fumarate crystal form II. The XRPD pattern of the crystal form is shown in FIG. 7, and its characteristic peak positions are shown in Table 10. According to an ion chromatography analysis, the fumarate ion content was 18.78%. A DSC profile of the crystal form shows endothermic peaks at 155.97 °C and 229.48 °C. A TGA profile of the crystal form shows a weight loss of 1.3% from 30 °C to 100 °C and a weight loss of 21.4% from 100 °C to 280 °C.

**Table 10. The peak positions of fumarate crystal form II**

| Peak No. | 2*θ* value [° or degrees] | d[Å] | Relative intensity% |
|---|---|---|---|
| 1 | 7.032 | 12.55978 | 49.3 |
| 2 | 9.426 | 9.37547 | 66.8 |
| 3 | 10.934 | 8.08549 | 32.7 |
| 4 | 14.573 | 6.07359 | 39.9 |
| 5 | 14.966 | 5.91481 | 91.9 |
| 6 | 15.556 | 5.69174 | 1.7 |
| 7 | 17.130 | 5.17225 | 57.5 |
| 8 | 17.720 | 5.00131 | 100.0 |
| 9 | 18.736 | 4.73226 | 24.5 |
| 10 | 19.949 | 4.44717 | 24.2 |
| 11 | 23.850 | 3.72783 | 32.2 |

### Example 15. Preparation of Fumarate Crystal Form III of Compound

100 mg of compound I was weighed out and dissolved in 5 mL of 1,4-dioxane, and 16.5 mg of fumaric acid was added. The mixture was stirred at room temperature for 16 h, and a solid precipitated and was dried to give a product.

According to an X-ray powder diffraction analysis, the product was defined as fumarate crystal form III. The XRPD pattern of the crystal form is shown in FIG. 8, and its characteristic peak positions are shown in Table 11. ¹H NMR nuclear magnetic resonance characterization: Compound I was salified with fumaric acid in a 1:1 ratio.

**Table 11. The peak positions of fumarate crystal form III**

| Peak No. | 2*θ* value [° or degrees] | D[Å] | Relative intensity% |
|---|---|---|---|
| 1 | 5.1420 | 17.173 | 34.8 |
| 2 | 6.0490 | 14.599 | 10.6 |
| 3 | 7.0590 | 12.513 | 10.0 |
| 4 | 8.4820 | 10.416 | 5.7 |
| 5 | 9.5570 | 9.247 | 24.5 |
| 6 | 10.3660 | 8.527 | 54.9 |
| 7 | 10.7270 | 8.240 | 28.5 |
| 8 | 11.3410 | 7.796 | 13.2 |
| 9 | 12.9100 | 6.852 | 7.7 |
| 10 | 13.1400 | 6.732 | 8.5 |
| 11 | 13.6830 | 6.466 | 3.8 |
| 12 | 16.1100 | 5.497 | 8.7 |
| 13 | 16.5020 | 5.368 | 8.5 |
| 14 | 16.6550 | 5.319 | 6.9 |
| 15 | 17.9480 | 4.938 | 59.7 |
| 16 | 18.4900 | 4.795 | 27.8 |
| 17 | 19.4290 | 4.565 | 23.8 |
| 18 | 20.5220 | 4.324 | 100.0 |
| 19 | 22.0790 | 4.023 | 9.2 |
| 20 | 22.1410 | 4.012 | 9.2 |
| 21 | 23.2050 | 3.830 | 20.0 |
| 22 | 25.3590 | 3.509 | 7.5 |

### Example 16. Preparation of Fumarate Crystal Form IV

20 mg of compound I was weighed out and dissolved in 1 mL of acetone, and 0.2 mL of a solution of fumaric acid (3.3 mg, 28.41 µmol) in acetone was added. The mixture was stirred at room temperature for 2.5 h, and a solid precipitated and was dried to give a product.

According to an X-ray powder diffraction analysis, the product was defined as fumarate crystal form IV. The XRPD pattern of the crystal form is shown in FIG. 9, and its characteristic peak positions are shown in Table 12. ¹H NMR nuclear magnetic resonance characterization: Compound I was salified with fumaric acid in a 1:1 ratio.

**Table 12. The peak positions of fumarate crystal form IV**

| Peak No. | 2*θ* value [° or degrees] | D[Å] | Relative intensity% |
|---|---|---|---|
| 1 | 6.211 | 14.218 | 64.3 |
| 2 | 10.300 | 8.582 | 100.0 |
| 3 | 11.050 | 8.001 | 48.1 |
| 4 | 12.458 | 7.100 | 25.7 |
| 5 | 15.757 | 5.620 | 43.8 |
| 6 | 21.255 | 4.177 | 47.7 |
| 7 | 22.179 | 4.005 | 38.7 |

### Example 17. Preparation of Fumarate Crystal Form V

100 mg of compound I was weighed out and dissolved in 2 mL of 1,4-dioxane, and 0.4 mL of a solution of fumaric acid (16.5 mg, 142.06 µmol) in 1,4-dioxane was added. The mixture was stirred at room temperature for 16 h, and a solid precipitated and was dried to give a product.

According to an X-ray powder diffraction analysis, the product was defined as fumarate crystal form V. The XRPD pattern of the crystal form is shown in FIG. 10, and its characteristic peak positions are shown in Table 13. A DSC profile of the crystal form shows endothermic peaks at 142.15 °C, 158.14 °C, and 232.14 °C. A TGA profile of the crystal form shows a weight loss of 8.10% from 30 °C to 140 °C and a weight loss of 1.78% from 140 °C to 190 °C. ¹H NMR nuclear magnetic resonance characterization: Compound I was salified with fumaric acid in a 1:1 ratio.

**Table 13. The peak positions of fumarate crystal form V**

| Peak No. | 2*θ* value [° or degrees] | D[Å] | Relative intensity% |
|---|---|---|---|
| 1 | 5.2560 | 16.801 | 25.3 |
| 2 | 6.2150 | 14.209 | 15.5 |
| 3 | 8.5450 | 10.339 | 10.6 |
| 4 | 9.9170 | 8.912 | 23.5 |
| 5 | 10.4840 | 8.431 | 96.6 |
| 6 | 10.9700 | 8.059 | 10.9 |
| 7 | 11.5950 | 7.626 | 23.4 |
| 8 | 13.2090 | 6.697 | 10.9 |
| 9 | 15.5890 | 5.680 | 21.9 |
| 10 | 16.0390 | 5.521 | 23.2 |
| 11 | 16.5170 | 5.363 | 8.8 |
| 12 | 17.0650 | 5.192 | 42.9 |
| 13 | 17.6290 | 5.027 | 12.5 |
| 14 | 18.5910 | 4.769 | 54.5 |
| 15 | 20.1290 | 4.408 | 28.6 |
| 16 | 21.0400 | 4.219 | 100.0 |
| 17 | 22.6710 | 3.919 | 22.1 |
| 18 | 23.2490 | 3.823 | 17.4 |
| 19 | 23.6880 | 3.753 | 28.5 |
| 20 | 25.0020 | 3.559 | 8.6 |
| 21 | 25.7530 | 3.457 | 6.1 |
| 22 | 28.1260 | 3.170 | 6.3 |

### Example 18. Preparation of L-Tartrate Crystal Form I

100 mg of compound I was weighed out and dissolved in 5 mL of 1,4-dioxane, and L-tartaric acid (21.5 mg, 143.25 µmol) was added. The mixture was stirred at room temperature for 16 h, and a solid precipitated and was dried to obtain a product.

According to an X-ray powder diffraction analysis, the product was defined as L-tartrate crystal form I. The XRPD pattern of the crystal form is shown in FIG. 11, and its characteristic peak positions are shown in Table 14. ¹H NMR nuclear magnetic resonance characterization: Compound I was salified with L-tartaric acid in a 1:1 ratio.

**Table 14. The peak positions of L-tartrate crystal form I**

| Peak No. | 2*θ* value [° or degrees] | D[Å] | Relative intensity% |
|---|---|---|---|
| 1 | 5.6900 | 15.520 | 18.7 |
| 2 | 6.9950 | 12.627 | 8.3 |
| 3 | 9.0580 | 9.755 | 52.2 |
| 4 | 9.5380 | 9.265 | 24.9 |
| 5 | 10.3530 | 8.537 | 10.6 |
| 6 | 10.7870 | 8.195 | 43.1 |
| 7 | 11.3890 | 7.763 | 13.5 |
| 8 | 13.7510 | 6.435 | 8.9 |
| 9 | 15.1230 | 5.854 | 4.4 |
| 10 | 16.0190 | 5.528 | 70.5 |
| 11 | 16.5900 | 5.339 | 5.0 |
| 12 | 17.2170 | 5.146 | 15.6 |
| 13 | 17.7290 | 4.999 | 82.6 |
| 14 | 18.0000 | 4.924 | 100.0 |
| 15 | 18.4150 | 4.814 | 35.1 |
| 16 | 19.0100 | 4.665 | 20.5 |
| 17 | 19.5940 | 4.527 | 22.1 |
| 18 | 20.0340 | 4.429 | 60.6 |
| 19 | 20.7230 | 4.283 | 49.5 |
| 20 | 21.5920 | 4.112 | 30.1 |
| 21 | 22.1390 | 4.012 | 11.9 |
| 22 | 22.4060 | 3.965 | 49.4 |
| 23 | 23.8530 | 3.727 | 23.1 |
| 24 | 25.2940 | 3.518 | 13.7 |
| 25 | 26.8250 | 3.321 | 22.6 |
| 26 | 27.5210 | 3.238 | 12.2 |
| 27 | 29.4540 | 3.030 | 3.0 |
| 28 | 30.9420 | 2.888 | 7.4 |
| 29 | 32.2340 | 2.775 | 4.5 |

### Example 19. Preparation of Succinate Crystal Form I

100 mg of compound I was weighed out and dissolved in 5 mL of 1,4-dioxane, and succinic acid (17 mg, 143.96 µmol) was added. The mixture was stirred at room temperature for 16 h, and a solid precipitated and was dried to obtain a product.

According to an X-ray powder diffraction analysis, the product was defined as succinate crystal form I. The XRPD pattern of the crystal form is shown in FIG. 12, and its characteristic peak positions are shown in Table 15. ¹H NMR nuclear magnetic resonance characterization: Compound I was salified with L-tartaric acid in a 1:1 ratio.

**Table 15. The peak positions of succinate crystal form I**

| Peak No. | 2*θ* value [° or degrees] | D[Å] | Relative intensity% |
|---|---|---|---|
| 1 | 5.6210 | 15.711 | 15.5 |
| 2 | 6.9240 | 12.757 | 12.5 |
| 3 | 8.3710 | 10.555 | 5.5 |
| 4 | 8.9810 | 9.839 | 28.9 |
| 5 | 9.5020 | 9.300 | 73.4 |
| 6 | 10.3010 | 8.581 | 32.7 |
| 7 | 10.7340 | 8.236 | 9.4 |
| 8 | 13.6790 | 6.468 | 14.1 |
| 9 | 15.0120 | 5.897 | 7.3 |
| 10 | 15.9530 | 5.551 | 50.9 |
| 11 | 16.9810 | 5.217 | 8.4 |
| 12 | 17.6490 | 5.021 | 72.3 |
| 13 | 17.9730 | 4.931 | 100.0 |
| 14 | 18.3180 | 4.839 | 27.9 |
| 15 | 19.0400 | 4.658 | 7.7 |
| 16 | 19.4870 | 4.552 | 16.8 |
| 17 | 19.8720 | 4.464 | 37.0 |
| 18 | 20.6500 | 4.298 | 76.5 |
| 19 | 21.5310 | 4.124 | 18.7 |
| 20 | 22.3300 | 3.978 | 62.0 |
| 21 | 23.2540 | 3.822 | 3.4 |
| 22 | 25.3490 | 3.511 | 7.7 |
| 23 | 25.4170 | 3.502 | 10.0 |
| 24 | 26.7750 | 3.327 | 13.0 |
| 25 | 27.0810 | 3.290 | 14.7 |
| 26 | 27.4450 | 3.247 | 5.4 |
| 27 | 31.1410 | 2.870 | 2.4 |
| 28 | 31.8610 | 2.806 | 2.9 |
| 29 | 35.5650 | 2.522 | 2.1 |
| 30 | 36.8520 | 2.437 | 2.8 |
| 31 | 44.9680 | 2.014 | 4.9 |

### Example 20. Preparation of D-Malate Crystal Form I

2 g of compound I was weighed out and dissolved in 60 mL of tetrahydrofuran, and 20 mL of a solution of D-malic acid (381 mg, 2.84 mmol) in tetrahydrofuran was added. The mixture was stirred at room temperature for 24 h in a dark place, and a solid precipitated and was dried *in vacuo* at 50 °C for 16 h to give a product.

According to an X-ray powder diffraction analysis, the product was defined as D-malate crystal form I. The XRPD pattern of the crystal form is shown in FIG. 13, and its characteristic peak positions are shown in Table 16. A DSC profile of the crystal form shows endothermic peaks at 122.76 °C, 142.13 °C, and 213.37 °C. A TGA profile of the crystal form shows a weight loss of 10.11% from 30 °C to 140 °C.

**Table 16. The peak positions of D-malate crystal form I**

| Peak No. | 2*θ* value [° or degrees] | D[Å] | Relative intensity% |
|---|---|---|---|
| 1 | 5.623 | 15.7053 | 27.6 |
| 2 | 6.951 | 12.7060 | 48.6 |
| 3 | 7.192 | 12.2821 | 20.4 |
| 4 | 8.960 | 9.8617 | 97.5 |
| 5 | 9.482 | 9.3201 | 70.5 |
| 6 | 10.269 | 8.6075 | 19.9 |
| 7 | 10.748 | 8.2245 | 68.8 |
| 8 | 11.338 | 7.7980 | 25.7 |
| 9 | 13.617 | 6.4975 | 16.5 |
| 10 | 15.934 | 5.5576 | 54.6 |
| 11 | 16.760 | 5.2855 | 18.3 |
| 12 | 17.587 | 5.0387 | 33.4 |
| 13 | 17.936 | 4.9415 | 100.0 |
| 14 | 18.218 | 4.8656 | 62.1 |
| 15 | 19.000 | 4.6671 | 13.6 |
| 16 | 19.746 | 4.4924 | 27.8 |
| 17 | 19.977 | 4.4410 | 34.3 |
| 18 | 20.607 | 4.3066 | 62.3 |
| 19 | 21.537 | 4.1227 | 33.6 |
| 20 | 22.266 | 3.9894 | 37.2 |
| 21 | 22.744 | 3.9066 | 9.8 |
| 22 | 23.927 | 3.7160 | 15.2 |
| 23 | 25.311 | 3.5160 | 10.9 |
| 24 | 26.711 | 3.3347 | 17.8 |
| 25 | 27.376 | 3.2552 | 20.3 |
| 26 | 28.461 | 3.1335 | 6.7 |

### Example 21. Preparation of Amorphous Sulfate

About 48 mg of compound I was weighed out and dissolved in 0.7 mL of 50% ethanol/ethyl acetate, and a sulfuric acid solution (2 mol/L, 38 µL) was added. The reaction mixture was slurried for 1 day and centrifuged, and drying was performed to give a product.

According to an X-ray powder diffraction analysis, no significant characteristic peaks were observed, which indicates that the product was an amorphous sulfate. According to an ion chromatography analysis, the sulfate ion content was 12.89%. A TGA profile of the amorphous form shows a weight loss of 4.4% from 30 °C to 100 °C.

### Example 22. Preparation of Amorphous Phosphate

About 48 mg of compound I was weighed out and dissolved in 0.7 mL of 50% ethanol/ethyl acetate, and a phosphoric acid solution (2 mol/L, 38 µL) was added. The reaction mixture was slurried for 1 day and centrifuged, and drying was performed to give a product.

According to an X-ray powder diffraction analysis, no significant characteristic peaks were observed, which indicates that the product was an amorphous phosphate. According to an ion chromatography analysis, the phosphate ion content was 13.77%. A TGA profile of the amorphous form shows a weight loss of 1.5% from 30 °C to 110 °C.

### Example 23. Preparation of Amorphous Tartrate

About 28 mg of compound I was weighed out and dissolved in 0.4 mL of 50% ethanol/ethyl acetate, and a tartaric acid solution (2 mol/L, 21 µL) was added. The reaction mixture was slurried for 1 day and centrifuged, and drying was performed to give a product.

According to an X-ray powder diffraction analysis, no significant characteristic peaks were observed, which indicates that the product was an amorphous tartrate. According to an ion chromatography analysis, the tartrate ion content was 9.87%. A TGA profile of the amorphous form shows a weight loss of 2.3% from 30 °C to 100 °C and a weight loss of 1% from 100 °C to 180 °C.

### Example 24. Preparation of Amorphous L-Malate

About 28 mg of compound I was weighed out and dissolved in 0.4 mL of 50% ethanol/ethyl acetate, and an L-malic acid solution (2 mol/L, 21 µL) was added. The reaction mixture was slurried for 1 day and centrifuged, and drying was performed to give a product.

According to an X-ray powder diffraction analysis, no significant characteristic peaks were observed, which indicates that the product was an amorphous L-malate. According to an ion chromatography analysis, the malate ion content was 10.25%. A TGA profile of the amorphous form shows a weight loss of 1.6% from 30 °C to 90 °C and a weight loss of 3.2% from 90 °C to 160 °C.

### Example 25. Preparation of Amorphous Citrate

About 28 mg of compound I was weighed out and dissolved in 0.4 mL of 50% ethanol/ethyl acetate, and a citric acid solution (0.5 mol/L, 84 µL) was added. The reaction mixture was slurried for 1 day and centrifuged, and drying was performed to give a product.

According to an X-ray powder diffraction analysis, the product was an amorphous citrate. A TGA profile of the amorphous form shows a weight loss of 2% from 30 °C to 100 °C.

### Experimental Example 1. Study on Influencing Factor Stability of Hydrochloride Crystal Form I

Samples of hydrochloride crystal form I were spread out in open vessels and subjected to 1-month stability tests under light exposure (4500 Lux), high temperature (40 °C and 60 °C), and high humidity (RH 75% and RH 92.5%) conditions.

**Table 17. The influencing factor stability of hydrochloride crystal form I**

| Conditions | Time (days) | Purity% | Crystal form |
|---|---|---|---|
| Initial | 0 | 99.0 | Hydrochloride crystal form I |
| 40°C | 7 | 98.6 | Hydrochloride crystal form I |
| | 14 | 98.4 | Hydrochloride crystal form I |
| | 30 | 97.9 | Hydrochloride crystal form I |
| 60°C | 7 | 98.2 | Hydrochloride crystal form I |
| | 14 | 97.6 | Hydrochloride crystal form I |
| | 30 | 96.8 | Hydrochloride crystal form I |
| 75% RH | 7 | 99.0 | Hydrochloride crystal form I |
| | 14 | 98.9 | Hydrochloride crystal form I |
| | 30 | 99.0 | Hydrochloride crystal form I |
| 93% RH | 7 | 99.0 | Hydrochloride crystal form I |
| | 14 | 98.9 | Hydrochloride crystal form I |
| | 30 | 98.7 | Hydrochloride crystal form I |
| 4500 Lux | 7 | 98.6 | Hydrochloride crystal form I |
| | 14 | 98.1 | Hydrochloride crystal form I |
| | 30 | 97.8 | Hydrochloride crystal form I |

Conclusion: Standing under conditions other than the high temperature and light exposure conditions for 1 month, hydrochloride crystal form I exhibited good physical and chemical stabilities.

### Experimental Example 2. Study on Long-Term Accelerated Stability of Hydrochloride Crystal Form I

Hydrochloride crystal form I was left to stand under 25 °C/60% RH and 40 °C/75% RH conditions to test its stability.

**Table 18. The long-term/accelerated stability of hydrochloride crystal form I**

| Conditions | Time (days) | Purity% | Crystal form |
|---|---|---|---|
| Initial | 0 | 99.0 | Hydrochloride crystal form I |
| 25°C/60%RH | 7 | 99.0 | Hydrochloride crystal form I |
| | 14 | 99.0 | Hydrochloride crystal form I |
| | 30 | 99.0 | Hydrochloride crystal form I |
| | 60 | 99.0 | Hydrochloride crystal form I |
| 40°C/75%RH | 7 | 99.0 | Hydrochloride crystal form I |
| | 14 | 99.0 | Hydrochloride crystal form I |
| | 30 | 99.0 | Hydrochloride crystal form I |
| | 60 | 98.9 | Hydrochloride crystal form I |

| | | | |
|---|---|---|---|
| Conclusion: Standing under long-term accelerated conditions for 2 months, hydrochloride crystal form I exhibited good physical and chemical stabilities. | | | |

## Claims

1. A crystal form A of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the crystal form A has characteristic peaks at 14.2, 15.3, 16.1, 17.4, and 19.1, preferably at 14.2, 15.3, 16.1, 17.4, 19.1, 20.0, 20.9, and 22.3, and more preferably at 5.0, 6.0, 14.2, 15.3, 16.1, 17.4, 19.1, 20.0, 20.9, 22.3, 24.8, and 26.9; most preferably, the X-ray powder diffraction pattern expressed in terms of 2*θ* angles, which are diffraction angles, is shown in FIG. 2.

2. A pharmaceutically acceptable salt of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein the pharmaceutically acceptable salt is selected from the group consisting of hydrochloride, maleate, fumarate, L-tartrate, succinate, D-malate, L-malate, sulfate, phosphate, and citrate.

3. The pharmaceutically acceptable salt according to claim 2, wherein a chemical ratio of the (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione to acid is 3:1-1:3, preferably 2:1-1:2.

4. A preparation method for a pharmaceutically acceptable salt of (*S*)-3-(5-(4-((1-(4-((1R,2R)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, comprising a step of reacting (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione with an acid, wherein the acid is selected from the group consisting of hydrochloric acid, maleic acid, fumaric acid, L-tartaric acid, succinic acid, D-malic acid, L-malic acid, sulfuric acid, phosphoric acid, and citric acid.

5. A hydrochloride crystal form I of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the hydrochloride crystal form I has characteristic peaks at 13.8, 15.9, 19.0, 20.1, and 22.8, preferably at 13.8, 15.9, 16.9, 17.9, 19.0, 20.1, 20.6, and 22.8, and more preferably at 10.8, 13.8, 15.9, 16.9, 17.9, 19.0, 20.1, 20.6, 22.8, 25.2, and 26.5; most preferably, the X-ray powder diffraction pattern expressed in terms of 2*θ* angles, which are diffraction angles, is shown in FIG. 3.

6. A hydrochloride crystal form II of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the hydrochloride crystal form II has characteristic peaks at 13.2, 17.1, 19.7, 20.6, 22.8, and 25.0, preferably at 10.4, 13.2, 14.5, 15.1, 17.1, 19.7, 20.6, 22.8, 25.0, and 26.1, and more preferably at 5.6, 10.4, 13.2, 13.8, 14.5, 15.1, 17.1, 19.7, 20.6, 22.8, 23.3, 25.0, and 26.1; most preferably, the X-ray powder diffraction pattern expressed in terms of 2*θ* angles, which are diffraction angles, is shown in FIG. 4.

7. A maleate crystal form I of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the maleate crystal form I has characteristic peaks at 5.6, 8.8, 9.4, 10.2, 10.7, and 18.0, preferably at 5.6, 7.0, 8.8, 9.4, 10.2, 10.7, 15.9, 18.0, 20.6, and 22.3, and more preferably at 5.6, 7.0, 8.4, 8.8, 9.4, 10.2, 10.7, 11.3, 15.9, 16.8, 18.0, 20.6, 21.5, 22.3, and 23.9; most preferably, the X-ray powder diffraction pattern expressed in terms of 2*θ* angles, which are diffraction angles, is shown in FIG. 5.

8. A fumarate crystal form I of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the fumarate crystal form I has characteristic peaks at 6.8, 9.6, 10.5, 17.6, 18.2, and 21.1, preferably at 5.7, 6.8, 9.6, 10.5, 16.0, 17.6, 18.2, 19.8, and 21.1, and more preferably at 5.7, 6.8, 8.9, 9.6, 10.5, 11.3, 16.0, 17.6, 18.2, 19.8, 21.1, and 22.2; most preferably, the X-ray powder diffraction pattern expressed in terms of 2*θ* angles, which are diffraction angles, is shown in FIG. 6.

9. A fumarate crystal form II of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the fumarate crystal form II has characteristic peaks at 7.0, 9.4, 15.0, 17.1, 17.7, and 18.7, preferably at 7.0, 9.4, 10.9, 14.6, 15.0, 17.1, 17.7, 18.7, 19.9, and 23.9; more preferably, the X-ray powder diffraction pattern expressed in terms of 2*θ* angles, which are diffraction angles, is shown in FIG. 7.

10. A fumarate crystal form III of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the fumarate crystal form III has characteristic peaks at 5.1, 9.6, 10.4, 17.9, 18.5, and 20.5, preferably at 5.1, 6.0, 9.6, 10.4, 17.9, 18.5, 19.4, 20.5, and 23.2, and more preferably at 5.1, 6.0, 7.1, 9.6, 10.4, 11.3, 17.9, 18.5, 19.4, 20.5, and 23.2; most preferably, the X-ray powder diffraction pattern expressed in terms of 2*θ* angles, which are diffraction angles, is shown in FIG. 8.

11. A fumarate crystal form IV of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the fumarate crystal form IV has characteristic peaks at 6.2, 10.3, 11.1, 12.5, 21.3, and 22.2; preferably, the X-ray powder diffraction pattern expressed in terms of 2*θ* angles, which are diffraction angles, is shown in FIG. 9.

12. A fumarate crystal form V of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the fumarate crystal form V has characteristic peaks at 10.5, 17.1, 18.6, 20.1, 21.0, and 23.7, preferably at 5.3, 9.9, 10.5, 11.6, 16.0, 17.1, 18.6, 20.1, 21.0, and 23.7, and more preferably at 5.3, 6.2, 9.9, 10.5, 11.6, 15.6, 16.0, 17.1, 18.6, 20.1, 21.0, 22.7, 23.2, and 23.7; most preferably, the X-ray powder diffraction pattern expressed in terms of 2*θ* angles, which are diffraction angles, is shown in FIG. 10.

13. An L-tartrate crystal form I of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the L-tartrate crystal form I has characteristic peaks at 9.1, 16.0, 17.7, 18.0, 20.0, and 20.7, preferably at 9.1, 9.5, 10.8, 16.0, 17.7, 18.0, 20.0, 20.7, 21.6, and 22.4, and more preferably at 5.7, 9.1, 9.5, 10.8, 16.0, 17.2, 17.7, 18.0, 19.6, 20.0, 20.7, 21.6, 22.4, and 23.9; most preferably, the X-ray powder diffraction pattern expressed in terms of 2*θ* angles, which are diffraction angles, is shown in FIG. 11.

14. A succinate crystal form I of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the succinate crystal form I has characteristic peaks at 9.5, 16.0, 17.6, 18.0, 19.9, 20.7, and 22.3, preferably at 9.0, 9.5, 10.3, 16.0, 17.6, 18.0, 19.9, 20.7, 21.5, and 22.3, and more preferably at 5.6, 6.9, 9.0, 9.5, 10.3, 10.7, 13.7, 16.0, 17.6, 18.0, 19.9, 20.7, 21.5, and 22.3; most preferably, the X-ray powder diffraction pattern expressed in terms of 2*θ* angles, which are diffraction angles, is shown in FIG. 12.

15. A D-malate crystal form I of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione, wherein an X-ray powder diffraction pattern expressed in terms of 2*θ* angles, which are diffraction angles, of the D-malate crystal form I has characteristic peaks at 9.0, 9.5, 10.7, 15.9, 17.9, 18.2, and 20.6, preferably at 7.0, 9.0, 9.5, 10.7, 15.9, 17.9, 18.2, 20.0, 20.6, and 22.3, and more preferably at 5.6, 7.0, 9.0, 9.5, 10.7, 15.9, 17.6, 17.9, 18.2, 20.0, 20.6, 21.5, and 22.3; most preferably, the X-ray powder diffraction pattern expressed in terms of 2*θ* angles, which are diffraction angles, is shown in FIG. 13.

16. The crystal forms according to claims 1 and 5-15, wherein the 2*θ* angles have a margin of error of ±0.2.

17. A pharmaceutical composition, comprising the following components:
i) the crystal form A of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 1, or the pharmaceutically acceptable salt of (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 2, or the crystal form according to any one of claims 5-15; and
ii) one or more pharmaceutically acceptable excipients.

18. A method for preparing a pharmaceutical composition, comprising the following step: a step of mixing the crystal form A of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 1, or the pharmaceutically acceptable salt of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 2, or the crystal form according to any one of claims 5-15 with a pharmaceutically acceptable excipient.

19. Use of the crystal form A of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 1, or the pharmaceutically acceptable salt of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 2, or the crystal form according to any one of claims 5-15, or the composition according to claim 17 in the preparation of a medicament for treating or preventing a disorder that is treatable by degradation of a target protein that binds to a targeting ligand.

20. Use of the crystal form A of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 1, or the pharmaceutically acceptable salt of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 2, or the crystal form according to any one of claims 5-15, or the composition according to claim 17 in the preparation of a medicament for treating or preventing a disorder that is treatable by *in vivo* binding to cereblon, wherein preferably, the disorder is abnormal cell proliferation, a tumor, an immune disease, diabetes, a cardiovascular disease, an infectious disease, and an inflammatory disease; more preferably, the disorder is a tumor or an infectious disease.

21. Use of the crystal form A of (*S*)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 1, or the pharmaceutically acceptable salt of (S)-3-(5-(4-((1-(4-((1*R*,2*R*)-6-hydroxy-2-isobutyl-1,2,3,4-tetrahydronaphthalen-1-yl)phenyl)piperidin-4-yl)methyl)piperazin-1-yl)-1-oxoisoindolin-2-yl)piperidine-2,6-dione according to claim 2, or the crystal form according to any one of claims 5-15, or the composition according to claim 17 in the preparation of a medicament for treating or preventing an estrogen receptor-mediated or -dependent disease or disorder.
